(19) Europäisches Patentamt
European Patent Office
Office européen des brevets

(11) Publication number: 0 176 916
A2

# (12) EUROPEAN PATENT APPLICATION

(21) Application number: 85112054.3

(22) Date of filing: 24.09.85

(51) Int. Cl.⁴: C 12 N 15/00
C 07 H 21/04

A request for correction of the description has been filed pursuant to Rule 88 EPC. A decision on the request will be taken during the proceedings before the Examining Division.

(30) Priority: 25.09.84 GB 8424198

(43) Date of publication of application: 09.04.86 Bulletin 86/15

(84) Designated Contracting States: BE CH DE FR GB IT LI NL SE

(71) Applicant: Fujisawa Pharmaceutical Co., Ltd.
3, Doshomachi 4-chome Higashi-ku
Osaka-shi, Osaka 541(JP)

(72) Inventor: Ueda, Ikuo
No. 1-9-9, Kukuchinishimachi
Toyonaka-shi Osaka-fu, 560(JP)

(72) Inventor: Niwa, Mineo
No. 7-15 Kirinokuchi Kamiuenocho
Mukoo-shi Kyoto-fu, 617(JP)

(72) Inventor: Saito, Yoshimasa
No. 5-14-10 Higashitokiwadai Toyono-cho
Toyono-gun Osaka-fu, 563-01(JP)

(72) Inventor: Sato, Susumu
No. 7-9-4 Higashitokiwadai Toyono-cho
Toyono-gun Osaka-fu, 563-01(JP)

(72) Inventor: Ono, Hiroki
No. 3-402, Aoba 3-chome 12 shimamoto-cho
Mishima-gun Osaka-fu, 618(JP)

(72) Inventor: Kitaguchi, Tadashi
No. 1-9-9 Kukucghinishimachi
Amagasaki-shi Hyogo-ken, 661(JP)

(74) Representative: Türk, Dietmar, Dr. rer. nat. et al,
Patentanwälte Türk & Gille Brucknerstrasse 20
D-4000 Düsseldorf 13(DE)

## (54) Process for production of gamma-interferon.

(57) It is a gene coding for gamma-interferon disclosed in which the gene comprises a novel sequence. The gene is produced by linking the gene coding for IFN LH with a gene coding for IFN-RH.

EP 0 176 916 A2

Fig. 1 Building up of oligonucleotides from smaller units by successive coupling reactions (1/4)

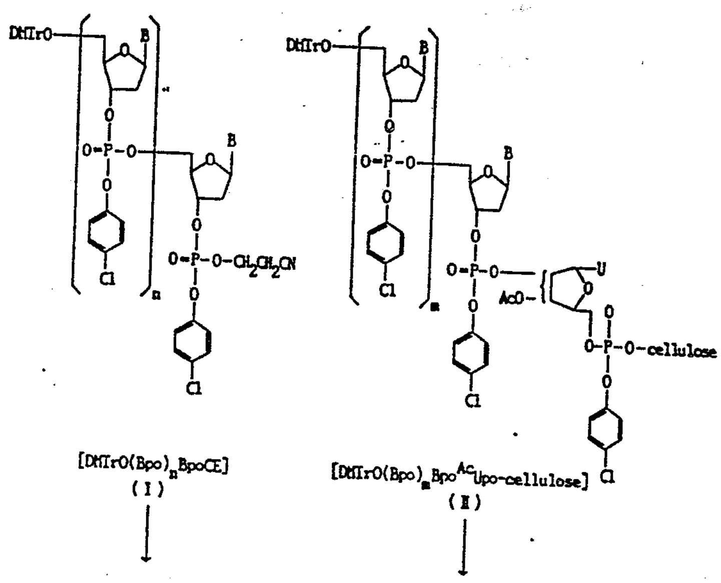

[DMTrO(Bpo)$_n$BpoCE]
(I)

[DMTrO(Bpo)$_m$Bpo$^{Ac}$Upo-cellulose]
(II)

Fig. 1 (continued) Building up of oligonucleotides from smaller units by successive coupling reactions (2/4)

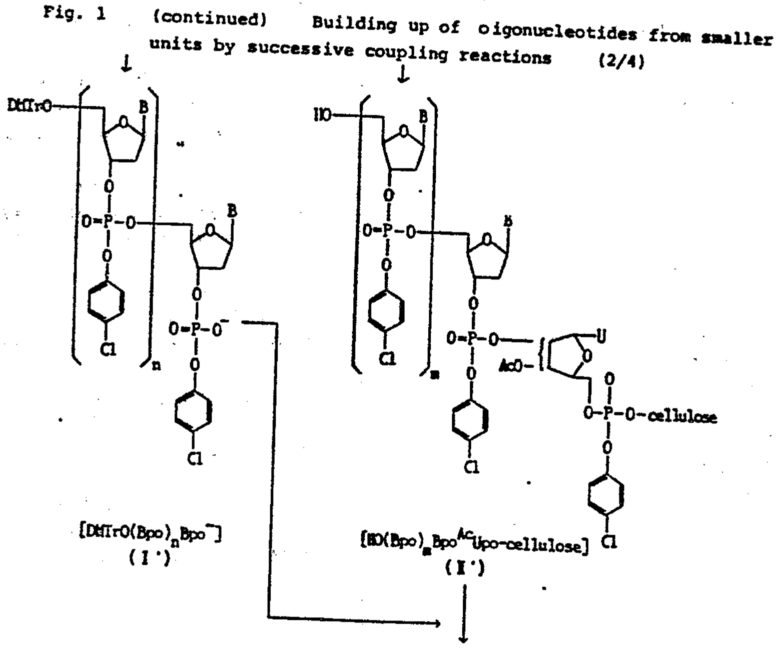

[DMTrO(Bpo)$_n$Bpo$^-$]
(I')

[HO(Bpo)$_m$Bpo$^{Ac}$Upo-cellulose]
(II')

Fig. 1 (continued) Building up of oligonucleotides from units by successive coupling reactions (3/4)

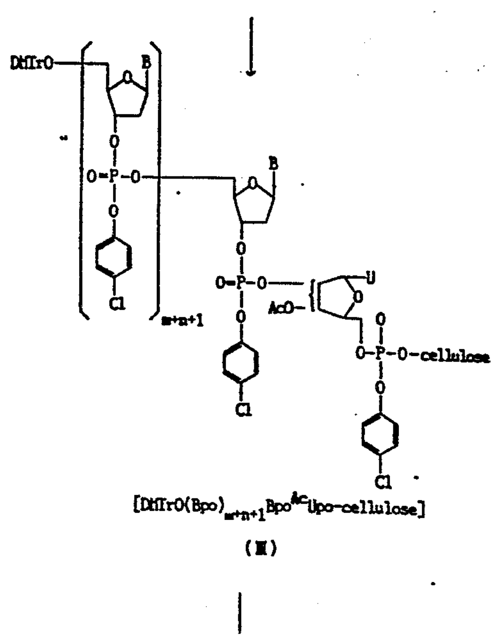

[DMTrO(Bpo)$_{m+n+1}$Bpo$^{Ac}$Upo-cellulose]
(III)

0176916

# PROCESS FOR PRODUCTION OF γ-INTERFERON

This invention relates to processes for production of γ-interferon (hereinafter referred to as IFN), to a gene coding for said IFN, to a plasmid containing said gene and to a host organism containing said plasmid, and to processes for production thereof.

IFN can be induced in lumphocytes by mitogens, alloantigens and other substances as well as being induced in sensitized lymphocytes by specific antigens. Biologically, IFN is a very interesting lymphokine with important immunoregulation properties. Moreover, IFN has been shown to have significant antitumor activity against mouse sarcomas. However, tests in the treatment of viral diseases and cancer have been severely restricted by its very limited availability.

There exists a need for a commercial production of IFN, and such a requirement stimulate the accomplishment of this invention.

It was perceived that the application of recombinant DNA and associated technologies would be a most effective way of producing large quantities of IFN.

IFN is known to consist of 146 amino acids in the following sequence:

1 10
Cys-Tyr-Cys-Gln-Asp-Pro-Tyr-Val-Lys-Glu-Ala-Glu-

20
Asn-Leu-Lys-Lys-Tyr-Phe-Asn-Ala-Gly-His-Ser-Asp-Val-

30
Ala-Asp-Asn-Gly-Thr-Leu-Phe-Leu-Gly-Ile-Leu-Lys-Asn-

40 50
Trp-Lys-Glu-Glu-Ser-Asp-Arg-Lys-Ile-Met-Gln-Ser-Gln-

60
Ile-Val-Ser-Phe-Tyr-Phe-Lys-Leu-Phe-Lys-Asn-Phe-Lys-

70
Asp-Asp-Gln-Ser-Ile-Gln-Lys-Ser-Val-Glu-Thr-Ile-Lys-

80 90
Glu-Asp-Met-Asn-Val-Lys-Phe-Phe-Asn-Ser-Asn-Lys-Lys-

100
Lys-Arg-Asp-Asp-Phe-Glu-Lys-Leu-Thr-Asn-Tyr-Ser-Val-

110
Thr-Asp-Leu-Asn-Val-Gln-Arg-Lys-Ala-Ile-His-Glu-Leu-

120
Ile-Gln-Val-Met-Ala-Glu-Leu-Ser-Pro-Ala-Ala-Lys-Thr-

130 140
Gly-Lys-Arg-Lys-Arg-Ser-Gln-Met-Leu-Phe-Gln-Gly-Arg-

146
Arg-Ala-Ser-Gln

The inventors of this invention succeeded in producing a large amount of IFN by using the following essential steps.

<u>Step 1</u>

A process for the production of a gene coding for IFN (hereinafter referred to as IFN gene) which comprises linking a gene coding for the left hand of IFN (hereinafter referred to as IFN LH) with a gene coding for the right hand of IFN (hereinafter referred to as IFN RH).

Suitable "gene" in the term "IFN gene" may include the ones as exemplified hereinafter and the like.

<u>Step 2</u>

A process for the production of an expression vector which comprises inserting a promoter gene and IFN gene into a plasmid.

Suitable "expression vector" may include plasmid pγtrp and the like.

Suitable "plasmid" may include pBR322 and the like.

Step 3

A process for the production of a transformant which comprises transforming a host organism with said expression vector.

Suitable "host organism" may include Escherichia (hereinafter referred to as E.) coli (e. g. E. coli HB101, etc.) and the like.

Step 4

A process for the production of IFN which comprises culturing said transformant in a suitable medium.

From the above amino acid sequence of IFN, a corresponding nucleotide sequence of IFN gene has been invented, subject to a number of specific non-obvious criteria. IFN gene has been constructed in two portions. One is a gene coding for the left hand of IFN (hereinafter referred to as IFN LH gene) and the other is a gene coding for the right hand of IFN (hereinafter referred to as IFN RH gene). The IFN LH gene is cloned by inserting it into a known plasmid, as a cloning vector and transformed to a host organism from which the plasmid

having IFN LH gene has been isolated. Thus obtained plasmid having IFN LH is used as a cloning vector of IFN RH gene. That is, IFN RH gene is inserted downstream of and adjacent to IFN LH gene, and transformed to a host organism , from which the plasmid having IFN gene is isolated. IFN gene is isolated from the plasmid having IFN gene. Thus obtained IFN gene is inserted into a plasmid specifically designed to maximize expression of the IFN gene under the control of a promoter.

Although the present invention is illustrated in detail hereinafter, the present invention is not limited thereto.

[1] Preparation and cloning of a IFN gene:

(1) Preparation of a IFN LH gene and a IFN RH gene:

The present invention also relates to a IFN LH gene and IFN RH gene.

From the above amino acid sequence, because of the diversity of the genetic code, it is possible to predict numerous nucleotide sequences which would code for the IFN LH gene or IFN RH gene.

In the inventive determination of an optimum sequence from the large number of possibilities, several non-obvious criteria have been observed. Firstly, trinucleotide codons should be used which are acceptable in a host organism to be used. Secondly, it should be desirable to have different restriction enzyme recognition sites at the terminal of the molecule so as to allow insertion into plasmid in a desired orientation.

Moreover, it should be decided to select sites which will be allowed to use well known cloning vectors.
Thirdly, the synthesis should not be unnecessarily complicated, and illegitimate cross-hybridization should be minimized in order to facilitate gene assembly, so that stable off-diagonal interactions might be avoided as far as possible.

Considering the above-mentioned criteria, particularly in consideration of the second criteria mentioned above, the following slightly longer sequence can be selected.

Structure of IFN LH gene:

IFN LH gene codes for the 1st to the 73rd amino acids of IFN.

As a suitable embodiment of this invention, EcoRI and BamHI sites was selected and introduced at the 5' and 3' ends, respectively, and further HindIII site was selected and introduced over lysine (58th amino acid) and leucine (59th amino acid) thereof.

Further, a methionine codon (ATG) was inserted upstream of and adjacent to the N-terminal amino acid codon of IFN, and three stop codons (TAA, TGA and TAG) were inserted downstream of and adjacent to the C-terminal codon.

The one of the preferred sequence selected for the coding for portion of the IFN LH gene can be shown as follows:

```
                              1
                     EcoRI Met Cys Tyr Cys Gln Asp Pro Tyr
     Coding:    5'-AATTC-ATG-TGT-TAC-TGC-CAG-GAC-CCA-TAT-
     Noncoding:     3'-G-TAC-ACA-ATG-ACG-GTC-CTG-GGT-ATA-

          10                                      20
Val Lys Glu Ala Glu Asn Leu Lys Lys Tyr Phe Asn Ala Gly
GTA-AAA-GAA-GCA-GAA-AAC-CTT-AAG-AAA-TAC-TTT-AAT-GCA-GGT-
CAT-TTT-CTT-CGT-CTT-TTG-GAA-TTC-TTT-ATG-AAA-TTA-CGT-CCA-

                                 30
His Ser Asp Val Ala Asp Asn Gly Thr Leu Phe Leu Gly Ile
CAT-TCA-GAT-GTA-GCG-GAT-AAT-GGA-ACT-CTT-TTC-TTA-GGC-ATT-
GTA-AGT-CTA-CAT-CGC-CTA-TTA-CCT-TGA-GAA-AAG-AAT-CCG-TAA-

                40
Leu Lys Asn Trp Lys Glu Glu Ser Asp Arg Lys Ile Met Gln
TTG-AAG-AAT-TGG-AAA-GAG-GAG-AGT-GAC-AGA-AAA-ATA-ATG-CAG-
AAC-TTC-TTA-ACC-TTT-CTC-CTC-TCA-CTG-TCT-TTT-TAT-TAC-GTC-

 50                                 HindIII 60
Ser Gln Ile Val Ser Phe Tyr Phe Lys Leu Phe Lys Asn Phe
AGC-CAA-ATT-GTC-TCC-TTT-TAC-TTC-AAG-CTT-TTC-AAA-AAC-TTT-
TCG-GTT-TAA-CAG-AGG-AAA-ATG-AAG-TTC-GAA-AAG-TTT-TTG-AAA-
```

70

Lys Asp Asp Gln Ser Ile Gln Lys Ser Val Stop Stop BamHI
AAG-GAT-GAC-CAG-AGC-ATC-CAA-AAG-AGT-GTG-TAA-TGA-TAG
TTC-CTA-CTG-GTC-TCG-TAG-GTT-TTC-TCA-CAC-ATT-ACT-ATCCTAG

Structure of IFN RH gene:

IFN RH gene codes for the 60th to the 146th amino acids of IFN. As a suitable embodiment of this invention, HindIII and BamHI sites was selected and introduced at the 5' and 3' end, respectively, and a stop codon (TAA) was inserted downstream of and adjacent to the C-teminal codon.

60
HindIII Phe Lys
Coding: 5'-AG-CTT-TTC-AAA-
Noncoding: 3'-A-AAG-TTT-

70
Asn Phe Lys Asp Asp Gln Ser Ile Gln Lys Ser Val Glu Thr
AAC-TTT-AAG-GAT-GAC-CAG-AGC-ATC-CAA-AAG-AGT-GTG-GAG-ACC-
TTG-AAA-TTC-CTA-CTG-GTC-TCG-TAG-GTT-TTC-TCA-CAC-CTC-TGG-

80
Ile Lys Glu Asp Met Asn Val Lys Phe Phe Asn Ser Asn Lys
ATC-AAG-GAA-GAC-ATG-AAT-GTC-AAG-TTT-TTC-AAT-AGC-AAC-AAA-
TAG-TTC-CTT-CTG-TAC-TTA-CAG-TTC-AAA-AAG-TTA-TCG-TTG-TTT-

90 100
Lys Lys Arg Asp Asp Phe Glu Lys Leu Thr Asn Tyr Ser Val
AAG-AAA-CGT-GAT-GAC-TTC-GAA-AAG-CTG-ACT-AAT-TAT-TCG-GTA-
TTC-TTT-GCA-CTA-CTG-AAG-CTT-TTC-GAC-TGA-TTA-ATA-AGC-CAT-

110

Thr Asp Leu Asn Val Gln Arg Lys Ala Ile His Glu Leu Ile

ACT-GAC-TTG-AAT-GTC-CAA-CGC-AAA-GCA-ATA-CAT-GAA-CTC-ATC-

TGA-CTG-AAC-TTA-CAG-GTT-GCG-TTT-CGT-TAT-GTA-CTT-GAG-TAG-

120 130

Gln Val Met Ala Glu Leu Ser Pro Ala Ala Lys Thr Gly Lys

CAA-GTG-ATG-GCT-GAA-CTG-TCG-CCA-GCA-GCT-AAA-ACA-GGG-AAG-

GTT-CAC-TAC-CGA-CTT-GAC-AGC-GGT-CGT-CGA-TTT-TGT-CCC-TTC-

140

Arg Lys Arg Ser Gln Met Leu Phe Gln Gly Arg Arg Ala Ser

CGA-AAA-CGT-AGT-CAG-ATG-CTG-TTT-CAA-GGT-CGA-AGA-GCA-TCC-

GCT-TTT-GCA-TCA-GTC-TAC-GAC-AAA-GTT-CCA-GCT-TCT-CGT-AGG-

Gln stop BamHI

CAG-TAA-G

GTC-ATT-CCTAG

In the sequence in this specification A, G, C and T mean the formula:

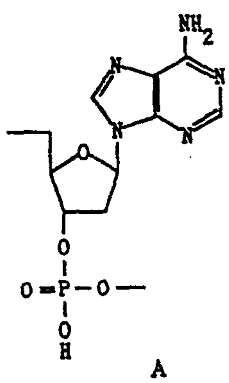
A

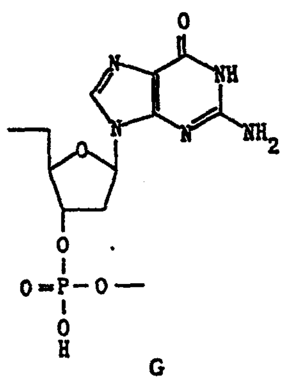
G

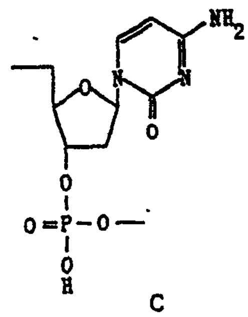
C

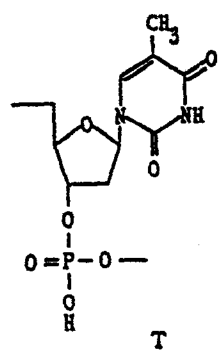
T

respectively, and

5'-terminal A, G, C and T mean the formula:

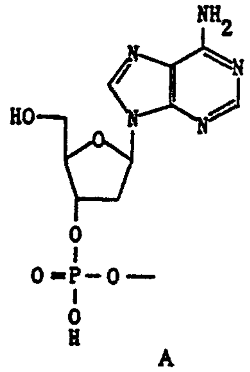
A
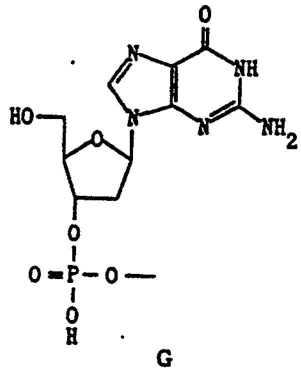
G
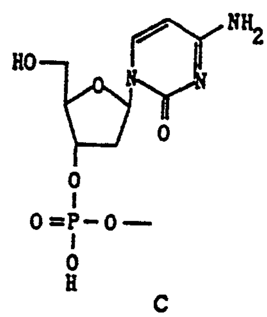
C
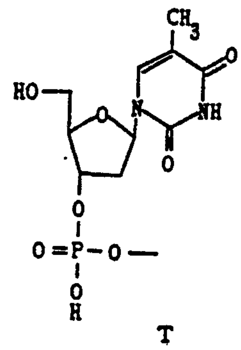
T

respectively, and

3'-terminal A, G, C and T mean the formula:

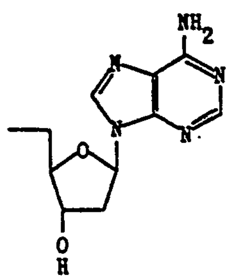
A
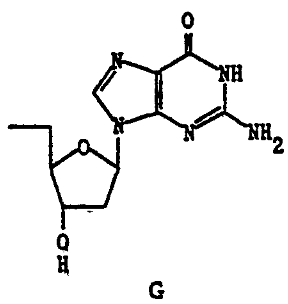
G
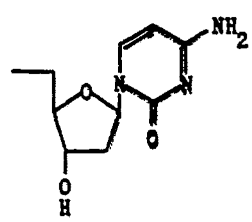
C
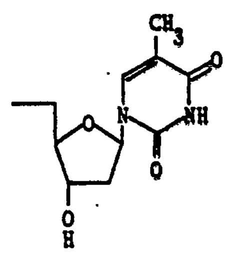
T

respectively.

The present invention also relates to a process for the production of such a gene characterized in that it comprises hybridization and ligation of a number of the corresponding oligonucleotide blocks.

A sub-unit of such a sequence, for example, a coding portion of IFN LH gene or IFN RH gene, also constitutes an aspect of the present invention.

(a) Synthesis of oligonucleotides:

It was in fact decided to synthesize a molecule having the above expanded sequence by making 62 synthetic oligonucleotides, which will hybridized and ligated in pre-determined stage to give the double-stranded nucleotide sequence, mentioned above.

In the specification about the synthesis of oligonucleotides in this specification, following abbreviations are used.

Ap, Gp, Cp and Tp mean the formula:

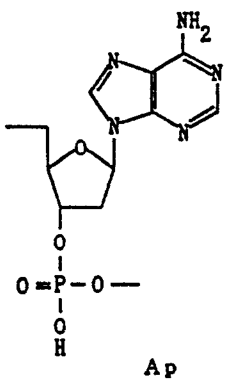
Ap

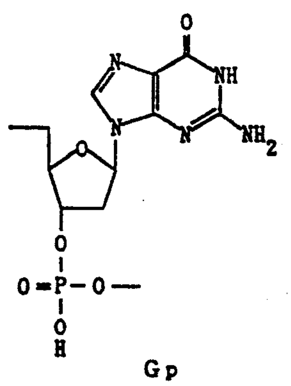
Gp

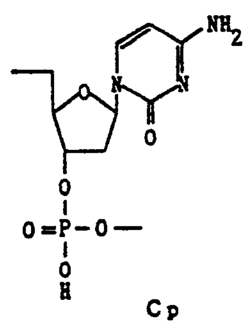
Cp

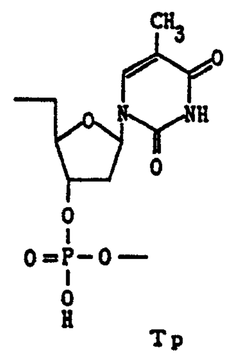
Tp

respectively, and

3'-teminal A, G, C and T mean the formula:

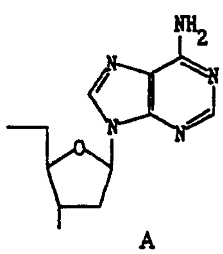
A

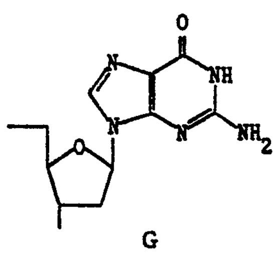
G

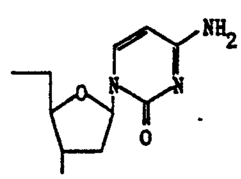
C

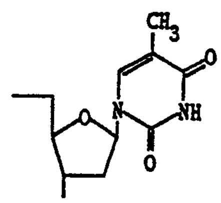
T

respectively, and

$A^{Bz}po$, $G^{iB}po$, $C^{Bz}po$, Tpo and ${}^{Ac}Upo$ mean the formula:

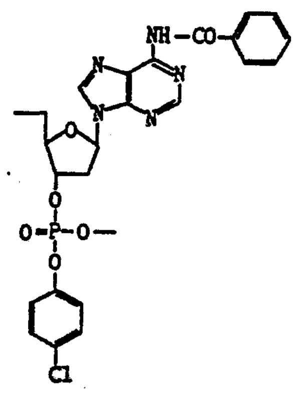

$A^{Bz}po$

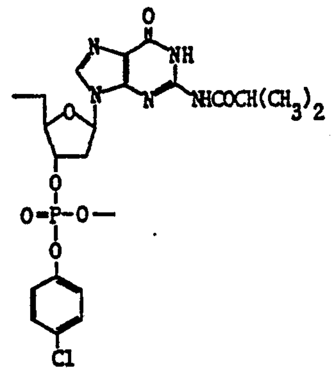

$G^{iB}po$

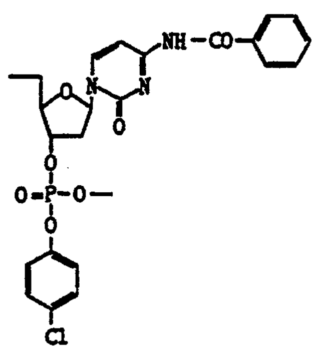

$C^{Bz}po$

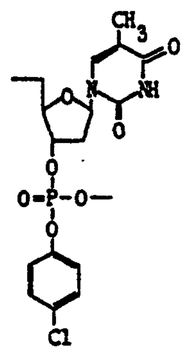

Tpo

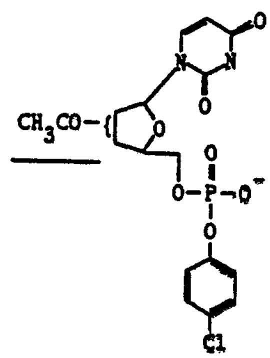

${}^{Ac}Upo$

respectively, and

DMTr is dimethoxytrityl,
B is a base selected from adeninyl, guaninyl, cytosinyl and thyminyl (for convenience, protecting group are not shown),
U is uracyl,
Ac is acetyl,
m is an integer of 1 or 2, and
n is an integer of 1 to 12.

The oligonucleotides are as follows:

(1) HOApApTpTpCpApTpGpTpGpTpTOH (A1)

(2) HOApCpTpGpCpCpApGpGpApCpCpCpApTOH (A2)

(3) HOApTpGpTpApApApApGpApApGpCpApGOH (A3)

(4) HOTpGpGpCpApGpTpApApCpApCpApTpGOH (A4)

(5) HOTpTpTpApCpApTpApTpGpGpGpTpCpCOH (A5)

(6) HOApApGpGpTpTpTpTpCpTpGpCpTpTpCpTOH (A6)

(7) HOApApApApCpCpTpTpApApGpApApApTpAOH (B1)

(8) HOCpTpTpTpApApTpGpCpApGpGpTpCpAOH (B2)

(9) HOTpTpCpApGpApTpGpTpApGpCpGpGpAOH (B3)

(10) HOApTpTpApApApGpTpApTpTpTpCpTpTOH (B4)

(11) HOApTpCpTpGpApApTpGpApCpCpTpGpCOH (B5)

(12) HOTpTpCpCpApTpTpApTpCpCpGpCpTpApCOH (B6)

(13) HOTpApApTpGpGpApApCpTpCpTpTpTpTpCOH (C1)

(14) HOTpTpApGpGpCpApTpTpTpTpGpApApGOH (C2)

(15) HOApApTpTpGpGpApApApGpApGpGpApGOH (C3)

(16) HOTpGpCpCpTpApApGpApApApApGpApGOH (C4)

(17) HOTpCpCpApApTpTpCpTpTpCpApApApAOH (C5)

(18) HOCpTpGpTpCpApCpTpCpTpCpCpTpCpTpTOH (C6)

(19) HOApGpTpGpApCpApGpApApApApApTpAOH (D1)

(20) HOApTpGpCpApGpApGpCpCpApApApTpTOH (D2)

(21) HOGpTpCpTpCpCpTpTpTpTpApCpTpTOH (D3)

(22) HOCpTpCpTpGpCpApTpTpApTpTpTpTpTOH (D4)

(23) HOApGpGpApGpApCpApApTpTpTpGpGOH (D5)

(24) HOApApApGpCpTpTpGpApApGpTpApApAOH (D6)

(25) HOCpApApGpCpTpTpTpTpCpApApApApAOH (E1)

(26) HOCpTpTpTpApApGpGpApTpGpApCpCpAOH (E2)

(27) HOGpApGpCpApTpCpCpApApApApGpApGOH (E3)

(28) HOCpCpTpTpApApApGpTpTpTpTpTpGpAOH (E4)

(29) HOGpGpApTpGpCpTpCpTpGpGpTpCpApTOH (E5)

(30) HOTpCpTpCpCpApCpApCpTpCpTpTpTpTOH (E6)

(31) HOTpGpTpGpGpApGpApCpCpApTpCpApAOH (F1)

(32) HOGpGpApApGpApCpApTpGpApApTpGpTOH (F2)

(33) HOCpApApGpTpTpTpTpTpCpApApTpApGOH (F3)

(34) HOTpGpTpCpTpTpCpCpTpTpGpApTpGpGOH (F4)

(35) HOApApApApCpTpTpGpApCpApTpTpCpAOH (F5)

(36) HOTpTpTpTpGpTpTpGpCpTpApTpTpGpAOH (F6)

(37) HOCpApApCpApApApApApGpApApApCpGOH (G1)

(38) HOTpGpApTpGpApCpTpTpCpGpApApApAOH (G2)

(39) HOGpCpTpGpApCpTpApApTpTpApTpTpCOH (G3)

(40) HOApGpTpCpApTpCpApCpGpTpTpTpCpTOH (G4)

(41) HOTpApGpTpCpApGpCpTpTpTpTpCpGpAOH (G5)

(42) HOCpApGpTpTpApCpCpGpApApTpApApTOH (G6)

(43) HOGpGpTpApApCpTpGpApCpTpTpGpApAOH (H1)

(44) HOTpGpTpCpCpApApCpGpCpApApApGpCOH (H2)

(45) HOApApTpApCpApTpGpApApCpTpCpApTpCOH (H3)

(46) HOGpTpTpGpGpApCpApTpTpCpApApGpTOH (H4)

(47) HOCpApTpGpTpApTpTpGpCpTpTpTpGpCOH (H5)

(48) HOApTpCpApCpTpTpGpGpApTpGpApGpTpTOH (H6)

(49) HOCpApApGpTpGpApTpGpGpCpTpGpApAOH (I1)

(50) HOCpTpGpTpCpGpCpCpApGpCpApGpCpTOH (I2)

(51) HOApApApApCpApGpGpGpApApGpCpGpAOH (I3)

(52) HOGpGpCpGpApCpApGpTpTpCpApGpCpCOH (I4)

(53) HOCpCpTpGpTpTpTpTpApGpCpTpGpCpTOH (I5)

(54) HOCpTpApCpGpTpTpTpTpCpGpCpTpTpCOH (I6)

(55) HOApApApCpGpTpApGpTpCpApGpApTpGpCOH (J1)

(56) HOTpGpTpTpTpCpApApGpGpTpCpGpApAOH (J2)

(57) HOGpApGpCpApTpCpCpCpApGpTpApApGOH (J3)

(58) HOTpGpApApApCpApGpCpApTpCpTpGpAOH (J4)

(59) HOGpApTpGpCpTpCpTpTpCpGpApCpCpTOH (J5)

(60) HOGpApTpCpCpTpTpApCpTpGpGOH (J6)

(61) HOTpGpTpGpTpApApTpGpApTpApGOH (L1)

(62) HOTpApCpApCpApCpTpCpTpTpTpTOH (L2)

(63) HOGpApTpCpCpTpApTpCpApTOH (L3)

The methods of building up oligonucleotides from smaller units by successive coupling reactions.

The successive coupling reaction is shown in Fig. 1.

Mono(or di, or tri)mer (I) can be prepared by the Hirose's method [T. Hirose, PROTEIN, NUCLEIC ACID AND ENZYME ISSN, 25, 225(1980), published in Japan], and coupling can be carried out on cellulose by a phosphotriester method [R. Crea et al, Nucleic Acid

Research 8, 2331(1980) and M. L. Duckworth et al, Nucleic Acid Research, 9, 1691(1981)].

Particularly, the synthetic methods will now be illustrated with reference to the synthesis of the hexadecanucleotide HOApTpCpApCpTpTpGpGpApTpGpApGpTpTOH (H6) described in Example 1. The flow chart of the synthesis of the hexadecanucleotide H6 is shown in Fig. 2.

(b) Hybridization and ligation of chemically synthesized oligonucleotide:

(i) IFN LH gene:

The oligonucleotides are hybridized and ligated in a series of steps, in order to minimize the possibilities for undesirable interactions as shown in Fig. 3. In Fig. 3, an oligonucleotide is illustrated with —— or •—— ( • means 5'-phosphorylated end), and blocked oligonucleotides are illustrated, for example, •—^—— (—^— means ligated position). Ligation is conducted in the presence of T4 DNA ligase.

Oligonucleotides A1 to A6, B1 to B6, C1 to C6, D1, D2, D4 and D5, D3, E1, E2, D6, E4 and E5, E3, L1, L2 and L3 were hybridized and ligated to give blocks 1, 2, 3, 4, 5 and 6, respectively. Block 2 and Block 3, and Block 4 and Block 5 were hybridized and ligated to give Blocks 7 and 8, respectively. Blocks 1 and 7, and blocks 6 and 8 were hybridized and ligated to give an objective polynucleotide IFN LH gene.

(ii) IFN RH gene:

The oligonucleotides D3, D6, E1 to E6, F1 to F6, G1 to G6, H1 to H6, I1 to I6, J1 to J6 are hybridized and ligated by a similar manner to that of IFN LH gene. Thus obtained ligated mixture was cleaved with HindIII to give an objective polynucleotide IFN RH gene. This process is shown in Fig. 4.

IFN RH gene is prepared by a similar manner to that of IFN LH gene.

(2) Molecular cloning of the IFN gene:

In order to clone the IFN LH gene, it is inserted into an appropriate plasmid, cloning vector, having suitable enzyme recognition sites in which the IFN LH gene can be inserted.

As a suitable embodiment of this invention IFN LH gene synthesized for the expression in E. coli was inserted into a plasmid originated in E. coli (e. g. pBR322, etc.) and cloning was conduced.

For example, in case using a plasmid pBR322 having EcoRI and BamHI sites, as shown in Fig. 5, the plasmid was cleaved by EcoRI and BamHI. In this case the plasmid has ampicillin resistance code (it is indicated by Amp) on the longer fragment when cleaved by EcoRI and BamHI, and tetracycline resistance code (it is indicated by Tet) vanishes in consequence of the cleavage of BamHI site. The longer fragment of EcoRI, BamHI-cleaved plasmid pBR322 was purified by electroelution, hybridized and ligated with a large excess of the IFN LH gene using T4 DNA ligase. Thus obtained mixture was transformed into E. coli HB101. The plasmid was isolated from one of the obtained several ampicillin resistant and tetracycline sensitive transformants and confirmed to contain IFN LH gene by digestion with restriction enzyme and

electrophoresis. Thus obtained plasmid is named as plasmid pLH107.

Then, the plasmid pLH107was cleaved by HindIII and BamHI. In this case the plasmid has ampicillin resistance code (it is indicated by Amp) on the longer fragment when cleaved by HindIII and BamHI, and tetracycline resistance code (it is indicated by Tet) vanishes in consequence of the cleavage of BamHI site. The longer fragment of HindIII, BamHI-cleaved plasmid pLH107 was purified by electroelution, hybridized and ligated with a large excess of the IFN RH gene using T4 DNA ligase. Thus obtained mixture was transformed into E. coli HB101. The plasmid was isolated from one of the obtained several ampicillin resistant and tetracycline sensitive transformants and confirmed to contain IFN gene by digestion with restriction enzyme and electrophoresis. This process is shown in Fig. 5. Thus obtained plasmid is named as plasmid pγF-3.

[2] Preparation and cloning of a promoter gene :

To obtain IFN from a host organism, a promoter gene which had a certain structural gene was designed.

A promoter gene obtained by such a criteria is inserted into a plasmid, and then IFN gene is inserted therein in a style that the promoter gene is located upstream of and adjacent to the IFN gene.

(1) Preparation and cloning of a trp promoter I gene:

Structure of trp promoter I gene:

As a suitable embodiment of this invention, the following type of a promoter (hereinafter referred to trp promoter I) was planned.

EcoRI*
5'-AATTTGCCGACATCATAACGGTTCTGGCAAATATTCTGAAATGAGC-
3'-    ACGGCTGTAGTATTGCCAAGACCGTTTATAAGACTTTACTCG-

TGTTGACAATTAATCATCGAACTAGTTAACTAGTACGCAAGTTCACGTAAA-
ACAACTGTTAATTAGTAGCTTGATCAATTGATCATGCGTTCAAGTGCATTT-

EcoRI
AAGGGTATCG-3'
TTCCCATAGCTTAA-5'

Preparation and cloning trp promoter II:

To insert the trp promoter I described above in a correct direction into a plasmid, following type of a synthetic promoter, hereinafter referred to as trp promoter II, having a certain length of base pair chain following EcoRI site of trp promoter I and BamHI site at 3'-end was prepared.

It was in fact decided to synthesize a molecule having 163 bp by making 22 synthetic oligonucleotide blocks, which will be assemble by single-strand overlaps to give the complete double-stranded nucleotide sequence.

```
   EcoRI*
5'- AATTTGCCGACATCATAACGGTTCTGGCAAATATTCTGAAATGAGC-
3'-      ACGGCTGTAGTATTGCCAAGACCGTTTATAAGACTTTACTCG-

TGTTGACAATTAATCATCGAACTAGTTAACTAGTACGCAAGTTCACGTAAA-
ACAACTGTTAATTAGTAGCTTGATCAATTGATCATGCGTTCAAGTGCATTT-

          EcoRI
AAGGGTATCGAATTCATGGCTGGTTGTAAGAACTTCTTTTGGAAGACTTTC-
TTCCCATAGCTTAAGTACCGACCAACATTCTTGAAGAAAACCTTCTGAAAG-

                BamHI
ACTTCGTGTTGATAG-3'
TGAAGCACAACTATCCTAG-5'
```

(a) Synthesis of oligonucleotides:

Twenty-two oligonucleotide was further synthesized.

(1) HOApApTpTpTpGpCpCpGpApCpAOH (A)

(2) HOCpGpTpTpApTpGpApTpGpTpCpGpGpCpAOH (B)

(3) HOTpCpApTpApApCpGpGpTpTpCpTpGpGpCOH (C)

(4) HOGpApApTpApTpTpTpGpCpCpApGpApApCOH (D)

(5) HOApApApTpApTpTpCpTpGpApApApTpGpAOH (E)

(6) HOTpCpApApCpApGpCpTpCpApTpTpTpCpAOH (F)

(7) HOGpCpTpGpTpTpGpApCpApApTpTpApApTOH (G)

(8) HOGpTpTpCpGpApTpGpApTpTpApApTpTpGOH (H)

(9) HOCpApTpCpGpApApCpTpApGpTpTpApApCOH (I)

(10) HOGpCpGpTpApCpTpApGpTpTpApApCpTpAOH (J)

(11) HOTpApGpTpApCpGpCpApApGpTpTpCpApCOH (K)

(12) HOCpTpTpTpTpTpApCpGpTpGpApApCpTpTOH (L)

(13) HOGpTpApApApApApGpGpGpTpApTpCpGOH (M)

(14) HOApApTpTpCpGpApTpApCpCOH (N)

(15) HOApApTpTpCpApTpGpGpCpTOH (SA)

(16) HOGpGpTpTpGpTpApApGpApApCpTpTpCpTOH (SB)

(17) HOTpTpTpGpGpApApGpApCpTpTpTOH (SC)

(18) HOCpApCpTpTpCpGpTpGpTpTpGpApTpApGOH (SD)

(19) HOTpTpApCpApApCpCpApGpCpCpApTpGOH (SE)

(20) HOCpCpApApApApGpApApGpTpTpCOH (SF)

(21) HOCpGpApApGpTpGpApApApGpTpCpTpTOH (SG)

(22) HOGpApTpCpCpTpApTpCpApApCpAOH (SH)

The synthetic method will now be illustrated with reference to the synthesis of hexadecanucleotide HOApTpCpApCpTpTpGpGpApTpGpApGpTpTOH (H6) mentioned above.

(b) Ligation of chemically synthesized oligonucleotides:

The oligonucleotide A to N and SA to SH were hybridized and ligated according to a similar manner to that of a IFN LH gene as shown in Fig. 6.

(c) Molecular cloning of trp promoter II gene:

The trp promoter II gene was inserted into a plasmid. As a suitable embodiment of this invention, the trp promoter II was inserted into a plasmid pBR322 by cleaving the sites with EcoRI and BamHI as shown in Fig. 7. Thus obtained plasmid (trp promoter II vector) is named as plasmid pTrpEB7.

[3] Construction of expression vector of IFN gene:

IFN gene is inserted into a plasmid containing a promoter gene, and the recombinant plasmid is transformed into a host organism.

As a suitable embodiment of this invention, the following recombinant plasmid was established to express IFN gene in E. coli.

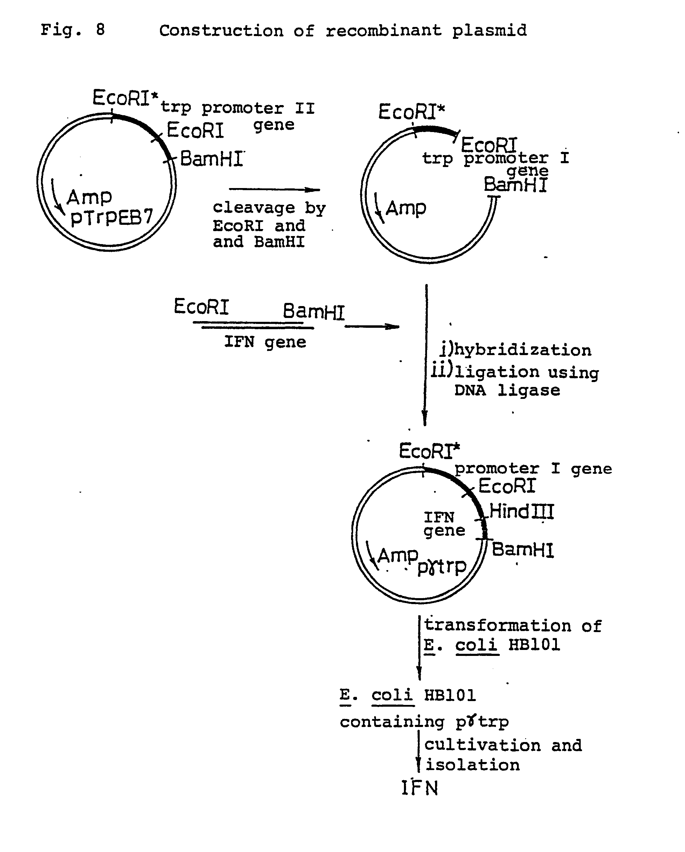

Trp promoter II plasmid pTrpEB7 was digested with EcoRI and BamHI, the resulted large fragment (4.1 kbp) was separated by agarose gel electrophoresis. On the other hand, the IFN gene was isolated from plasmid pγF-3, and ligated with the above promoter vector (4.1 kbp). The mixture was transformed into E. coli HB101. The plasmid was isolated from one of the obtained ampicillin

resistant and tetracycline sensitive transformants, and confirmed to contain structural protein LH gene by digestion with restriction enzyme and electrophoresis. Thus obtained plasmid is named as plasmid pγtrp. This process is shown in Fig. 8.

A gene coding for IFN is as follows:

```
                                 1
                     EcoRI Met Cys Tyr Cys Gln Asp Pro Tyr
    Coding:     5'-AATTC-ATG-TGT-TAC-TGC-CAG-GAC-CCA-TAT-
    Noncoding:     3'-G-TAC-ACA-ATG-ACG-GTC-CTG-GGT-ATA-


        10                                          20
Val Lys Glu Ala Glu Asn Leu Lys Lys Tyr Phe Asn Ala Gly
GTA-AAA-GAA-GCA-GAA-AAC-CTT-AAG-AAA-TAC-TTT-AAT-GCA-GGT-
CAT-TTT-CTT-CGT-CTT-TTG-GAA-TTC-TTT-ATG-AAA-TTA-CGT-CCA-


                                   30
His Ser Asp Val Ala Asp Asn Gly Thr Leu Phe Leu Gly Ile
CAT-TCA-GAT-GTA-GCG-GAT-AAT-GGA-ACT-CTT-TTC-TTA-GGC-ATT-
GTA-AGT-CTA-CAT-CGC-CTA-TTA-CCT-TGA-GAA-AAG-AAT-CCG-TAA-


                    40
Leu Lys Asn Trp Lys Glu Glu Ser Asp Arg Lys Ile Met Gln
TTG-AAG-AAT-TGG-AAA-GAG-GAG-AGT-GAC-AGA-AAA-ATA-ATG-CAG-
AAC-TTC-TTA-ACC-TTT-CTC-CTC-TCA-CTG-TCT-TTT-TAT-TAC-GTC-
```

50 HindIII 60
Ser Gln Ile Val Ser Phe Tyr Phe Lys Leu Phe Lys Asn Phe
AGC-CAA-ATT-GTC-TCC-TTT-TAC-TTC-AAG-CTT-TTC-AAA-AAC-TTT-
TCG-GTT-TAA-CAG-AGG-AAA-ATG-AAG-TTC-GAA-AAG-TTT-TTG-AAA-

70
Lys Asp Asp Gln Ser Ile Gln Lys Ser Val Glu Thr
AAG-GAT-GAC-CAG-AGC-ATC-CAA-AAG-AGT-GTG-GAG-ACC-
TTC-CTA-CTG-GTC-TCG-TAG-GTT-TTC-TCA-CAC-CTC-TGG-

80
Ile Lys Glu Asp Met Asn Val Lys Phe Phe Asn Ser Asn Lys
ATC-AAG-GAA-GAC-ATG-AAT-GTC-AAG-TTT-TTC-AAT-AGC-AAC-AAA-
TAG-TTC-CTT-CTG-TAC-TTA-CAG-TTC-AAA-AAG-TTA-TCG-TTG-TTT-

90 100
Lys Lys Arg Asp Asp Phe Glu Lys Leu Thr Asn Tyr Ser Val
AAG-AAA-CGT-GAT-GAC-TTC-GAA-AAG-CTG-ACT-AAT-TAT-TCG-GTA-
TTC-TTT-GCA-CTA-CTG-AAG-CTT-TTC-GAC-TGA-TTA-ATA-AGC-CAT-

110
Thr Asp Leu Asn Val Gln Arg Lys Ala Ile His Glu Leu Ile
ACT-GAC-TTG-AAT-GTC-CAA-CGC-AAA-GCA-ATA-CAT-GAA-CTC-ATC-
TGA-CTG-AAC-TTA-CAG-GTT-GCG-TTT-CGT-TAT-GTA-CTT-GAG-TAG-

120 130
Gln Val Met Ala Glu Leu Ser Pro Ala Ala Lys Thr Gly Lys
CAA-GTG-ATG-GCT-GAA-CTG-TCG-CCA-GCA-GCT-AAA-ACA-GGG-AAG-
GTT-CAC-TAC-CGA-CTT-GAC-AGC-GGT-CGT-CGA-TTT-TGT-CCC-TTC-

140
Arg Lys Arg Ser Gln Met Leu Phe Gln Gly Arg Arg Ala Ser
CGA-AAA-CGT-AGT-CAG-ATG-CTG-TTT-CAA-GGT-CGA-AGA-GCA-TCC-
GCT-TTT-GCA-TCA-GTC-TAC-GAC-AAA-GTT-CCA-GCT-TCT-CGT-AGG-

Gln stop BamHI
CAG-TAA-G
GTC-ATT-CCTAG

[4] Sequencing of IFN gene:

M13-dideoxy method can be used.

Sequence of IFN gene in plasmid pγtrp:

In sequencing of IFN gene was carried out using M13 dideoxy method developed by F. Sanger [F. Sanger et al, Proc. Natl. Acad. Sci. USA, 74, 5463(1977)].

For sequencing the IFN gene obtained by EcoRI-BamHI digestion of pγtrp was inserted into M13 vector (M13mp8RT) at EcoRI-BamHI site. The obtained plasmid was transformed into *E. coli* JM103 to give a white plaque on the plate containing Xgal (5-bromo-4-chloroindolylgalactoside). The single white plaque was incubated, followed by cell havest, to give phage particles. From the phage particles the single strand DNA (ssDNA) containing IFN gene was obtained, and then was analyzed according to the mannual of M13 dideoxy method. The results of sequencing of IFN gene were agreed with the designed IFN gene.

[5] Expression the IFN gene in a host organism:

For the expression of the IFN gene, thus obtained plasmid having a promoter gene and IFN gene is transformed into a host organism, and then the host organism having the plasmid is cultured in a suitable medium. IFN is characterized in the resulting culture broth.

(1) Expression of the IFN gene in E. coli using plasmid pγtrp:

An overnight culture of E. coli HB101 containing pγtrp in L broth was diluted in M9 medium lacking tryptophan, and the cells were incubated at 37°C for 3 hours under the condition of β-indoleacrylic acid induction. Detection of IFN production was carried out using a bioassay (anti-virus activity).

[6] Bioassay of IFN:

IFN activity was determined by the cytopathic effect (CPE) inhibition method with international standard LeuHuIFNα (National Institute of Health) as reference.

[7] Isolation of IFN:

The culture fluid was centrifuged to give a wet cell paste, and the cells were opened by sonication. The suspended mixture was centrifuged, the supernatant was examined by the bioassay of anti-virus activity to detect the activity of IFN. The IFN was detected a normal position on the SDS-PAGE. The activity of the obtained IFN diminished by acidic condition (pH 2.0). By the above evidence the production of IFN was characterized.

Thus obtained IFN is as follows:

```
1                                               10
Cys-Tyr-Cys-Gln-Asp-Pro-Tyr-Val-Cys-Glu-Ala-Glu-

                                20
Asn-Leu-Cys-Cys-Tyr-Phe-Asn-Ala-Gly-His-Ser-Asp-Val-

                30
Ala-Asp-Asn-Gly-Thr-Leu-Phe-Leu-Gly-Ilu-Leu-Lys-Asn-

    40                                          50
Trp-Lys-Glu-Glu-Ser-Asp-Arg-Lys-Ile-Met-Gln-Ser-Gln-

                                    60
Ile-Val-Ser-Phe-Tyr-Phe-Lys-Leu-Phe-Lys-Asn-Phe-Lys-

                    70
Asp-Asp-Gln-Ser-Ile-Gln-Lys-Ser-Val-Glu-Thr-Ile-Lys-

        80                                          90
Glu-Asp-Met-Asn-Val-Lys-Phe-Phe-Asn-Ser-Asn-Lys-Lys-

                                        100
Lys-Arg-Asp-Asp-Phe-Glu-Lys-Leu-Thr-Asn-Tyr-Ser-Val-

                        110
Thr-Asp-Leu-Asn-Val-Gln-Arg-Lys-Ala-Ile-His-Glu-Leu-

            120
Ile-Gln-Val-Met-Ala-Glu-Leu-Ser-Pro-Ala-Ala-Lys-Thr-

130                                         140
Gly-Lys-Arg-Lys-Arg-Ser-Gln-Met-Leu-Phe-Gln-Gly-Arg-
```

146
Arg-Ala-Ser-Gln

The following examples are given for the purpose of illustrating the present invention.

Example 1

Synthesis of HOApTpCpApCpTpTpGpGpApTpGpApGpTpTOH (H6)

(1) Synthesis of $DMTrOA^{Bz}poG^{iB}poTpoTpo^{Ac}Upo$-cellulose:

i) Preparation of $HOTpo^{Ac}Upo$-cellulose:

To a suspension of $DMTrOTpo^{Ac}Upo$-cellulose (84.0 mg, 3.57 μmole*) (prepared by R. Crea's method[1)]) in $MeOH/CHCl_3$ (1:9 v/v, 5.0 ml). $TCA/CHCl_3$ (2:8 w/v , 5.0 ml) was added under ice cooling, and the mixture was stirred at 0°C for 10 min. After being washed with $CHCl_3$ (2 ml) and MeOH (6.0 ml), successively, on the filter, the cellulose adduct ($HOTpo^{Ac}Upo$-cellulose) was dried, water being separated as the pyridine (2 ml) azeotrope.

* This value was calculated by monitoring the absorbance of a washing solution with $CHCl_3$ at 507 nm.

1) R. Crea et al, Nucleic Acid Res. 8, 2331(1980)

ii) Preparation of $DMTrOA^{Bz}poG^{iB}poTpo^-$:

$DMTrOA^{Bz}poG^{iB}poTpo$-CE (39.1 mg, 21.4 μmole) was treated with $Et_3N-CH_3CN$ (1:1 v/v, 5 ml) at room

temperature for 1 hr. The phosphodiester trimer (DMTrOA$^{Bz}$poG$^{iB}$poTpo$^{-}$) so obtained was dried, water being separated as the pyridine azeotrope (0.5 ml, 2 x 1 ml).

iii) Coupling:

The trimer (DMTrOA$^{Bz}$poG$^{iB}$poTpo$^{-}$) was mixed with the cellulose adduct (HOTpo$^{Ac}$Upo-cellulose) in a 10 ml round-bottom flask. The mixture was dried, water being separated as the pyridine azeotrope (2 x 1 ml) and finally resuspended in anhydrous pyridine (1 ml). Mesitylen sulfonyl nitrotriazolide (MSNT) (80.0 mg, 270 μmole) was added to the suspension and the mixture was stirred at room temperature for 1 hr. And then pyridine was added to the reaction vessel and cellulose adduct was recovered by centrifugation (3,000 rpm, 2 min).

iv) Acetylation of unreacted 5'hydroxyl groups:

The cellulose adduct obtained as above was suspended in a solution of pyridine-acetic anhydride (10:1 v/v, 5.5 ml) and stirred at room temperature for 30 min. The cellulose-product (56.0 mg) was obtained by repeated centrifugation (3,000 rpm, 2 min) in pyridine (5 ml), washing with MeOH (15 ml) and drying in vacuo at room temperature for 30 minutes. The cellulose adduct (DMTrOA$^{Bz}$poG$^{iB}$poTpoTpo$^{Ac}$Upo-Cellulose) can use for the next coupling step.

(2) Synthesis of DMTrOA$^{Bz}$poTpoG$^{iB}$poA$^{Bz}$poG$^{iB}$poTpoTpo-$^{Ac}$Upo-cellulose:

DMTrOA$^{Bz}$poTpoG$^{iB}$poA$^{Bz}$poG$^{iB}$poTpoTpo$^{Ac}$Upo-cellulose was synthesized from DMTrOA$^{Bz}$poG$^{iB}$poTpoTpo$^{Ac}$Upo-cellulose

(131.0 mg) and DMTrOA$^{Bz}$poTpoG$^{iB}$po-CE (39.1 mg) according to similar conditions as above.

(3) Synthesis of DMTrOTpoG$^{iB}$poG$^{iB}$poA$^{Bz}$poTpoG$^{iB}$poA$^{Bz}$po-G$^{iB}$poTpoTpo$^{Ac}$Upo-Cellulose:

DMTrOTpoG$^{iB}$poG$^{iB}$poA$^{Bz}$poTpoG$^{iB}$poA$^{Bz}$poG$^{iB}$poTpoT-po$^{Ac}$Upo-cellulose (105.8 mg) was synthesized from DMTrOA$^{Bz}$poTpoG$^{iB}$poA$^{Bz}$poG$^{iB}$poTpoTpo$^{Ac}$Upo-cellulose (123.0 mg) and DMTrOTpoG$^{iB}$poG$^{iB}$po-CE (38.7 mg) according to similar conditions.

(4) Synthesis of DMTrOA$^{Bz}$poC$^{Bz}$poTpoTpoG$^{iB}$poG$^{iB}$po-A$^{Bz}$po-TpoG$^{iB}$poA$^{Bz}$poG$^{iB}$poTpoTpo$^{Ac}$Upo-cellulose:

DMTrOA$^{Bz}$poC$^{Bz}$poTpoTpoG$^{iB}$poG$^{iB}$poA$^{Bz}$poTpoG$^{iB}$po-A$^{Bz}$poG$^{iB}$poTpoTpo$^{Ac}$Upo-cellulose (101.3 mg) was synthesized from DMTrOTpoG$^{iB}$poG$^{iB}$poA$^{Bz}$poTpoG$^{iB}$poA$^{Bz}$poG$^{iB}$poTpoTpo-$^{Ac}$Upo-cellulose (116.4 mg) and DMTrOA$^{Bz}$poC$^{Bz}$poTpo-CE (38.9 mg) according to similar conditions.

(5) Synthesis of DMTrOA$^{Bz}$poTpoC$^{Bz}$poA$^{Bz}$poC$^{Bz}$poTpo-TpoG$^{iB}$poG$^{iB}$poA$^{Bz}$poTpoG$^{iB}$poA$^{Bz}$poB$^{iB}$poTpoTpo $^{Ac}$Upo-cellulose:

DMTrOA$^{Bz}$poTpoC$^{Bz}$poA$^{Bz}$poC$^{Bz}$poTpoTpoG$^{iB}$poG$^{iB}$po-A$^{Bz}$poTpoG$^{iB}$poA$^{Bz}$poG$^{iB}$poTpoTpo$^{Ac}$Upo-cellulose (94.6 mg) was synthesized from DMTrOA$^{Bz}$poC$^{Bz}$poTpoTpoG$^{iB}$po-G$^{iB}$poA$^{Bz}$poTpoG$^{iB}$poA$^{Bz}$poG$^{iB}$poTpoTpo$^{Ac}$Upo-Cellulose (101.3 mg) and DMTrOA$^{Bz}$poTpoC$^{Bz}$po-CE (38.9 mg) under the similar conditions. At this final process, unreacted 5'-hydroxy group was not necessary to protect with an acetyl group.

(6) Synthesis of HOApTpCpApCpTpTpGpGpApTpGpApGpTpTOH:

DMTrOA$^{Bz}$poTpoC$^{Bz}$poA$^{Bz}$poC$^{Bz}$poTpoTpoG$^{iB}$poG$^{iB}$po A$^{Bz}$poTpoG$^{iB}$poA$^{Bz}$poG$^{iB}$poTpoTpo$^{Ac}$Upo-cellulose (94.6 mg) was treated with 0.5 M N,N,N',N'-tetramethylguanidinium pyridine 2-aldoximate (in dioxane-$H_2O$ (1:1 v/v, 1 ml)) at 20°C for 20 hrs in a sealed tube. To the reaction mixture 28% (w/w) aqueous ammonia (12 ml) was added, and the mixture was heated at 60°C for 2 hrs. The solid cellulose was removed by filtration and washed with water (10 ml). The filtrate and washed solution were evaporated to dryness, and the residue was treated with 80% aqueous acetic acid (25 ml) at room temperature for 15 mins. After removal of the solvents, the residue was dissolved in 0.1 M triethylammonium carbonate buffer (pH 7.5, 25 ml) was washed with diethylether (3 x 25 ml). Aqueous layer was evaporated to dryness and the residue was dissolved in 0.1 M triethylammonium carbonate buffer (pH 7.5, 2 mins) to yield crude HOApTpCpApCpTpTpGpGpApTpGpApGpTpTOH in the solution.

(7) Purification of HOApTpCpApCpTpTpGpGpApTpGpApGpTpTOH

i) First purification of the crude product was performed by column chromatography on Biogel P2 (24 x 2.6 cm ID). The fractions corresponding to the first eluted peak (50 mM $NH_4OAc$, 0.1 mM EDTA, 1 ml/min) were collected and freeze-dried to give the first purified product.

ii) Second purification of the first purified product was performed by HPLC on CDR-10 (25 cm x 4.6 mm ID) using a linear gradient of 1M $NH_4OAc$-10% (v/v) aqueous EtOH to 4.5 M $NH_4OAc$-10% (v/v) aqueous EtOH (80 min, 1 ml/min, 60°C) to give the second purified product. (Fig. 1)

iii) Third purification of the second purified product was performed by reverse phase HPLC (Rp-18-5μ(x77), 15 cm x 4mm ID) using a linear gradient of 0.1 M $NH_4OAc$ to 0.1 M $NH_4OAc$ 15% (v/v) aqueous $CH_3CN$ (40 min, 1.5 ml/min, room temperature) to give the final purified product (HOApTpCpApCpTpTpGpGpApTpGpApGpTpTOH). (Fig. 2)

(8) Analysis of oligonucleotide:
(HOApTpCpApCpTpTpGpGpApTpGpApTpTOH)

i) Digestion by phosphodiesterase:

The mixture of HOApTpCpApCpTpTpGpGpApTpGpApGpTpTOH (5 μg, 61.7 μl), 0.2 M $MgCl_2$ (10 μl), 0.2 M Tris-HCl (pH 8.5) (10 μl) and 0.1 mM EDTA in an aqueous solution (13.3 μl) was treated with phosphodiesterase (5 unit, 5 μl) at 37°C for 20 min, and then heated at 100°C for 2 min.

ii) Analysis by HPLC:

The oligonucleotide in the reaction mixture was analyzed by HPLC (CDR-10, 25 cm x 4.6 mm ID) using a linear gradient of water to 2.0 M $NH_4OAc$ (pH 3.4) (40 min, 1.5 ml/min, 60°C). From each peak area observed, its nucleotide composition was determined comparing with area of a standard sample.

Calcd: $pC_{OH}$ 2,000, $pA_{OH}$ 3,000, $pT_{OH}$ 6,000, $pG_{OH}$ 4,000
Observed: $pC_{OH}$ 1,886, $pA_{OH}$ 3,208, $pT_{OH}$ 5,949, $pG_{OH}$ 3,958

<u>Example 2</u>

Synthesis of oligonucleotides (A1, A2, A3, A4, A5, A6, B1, B2, B3, B4, B5, B6, C1, C2, C3, C4, C5, C6, D1, D2,

D3, D4, D5, D6, E1, E2, E3, E4, E5, E6, F1, F2, F3, F4, F5, F6, G1, G2, G3, G4, G5, G6, H1, H2, H3, H4, H5, H6, I1, I2, I3, I4, I5, I6, J1, J2, J3, J4, J5, J6, L1, L2, and L3):

Following oligonucleotides were prepared by a similar manner to that of H6 described in Example 1.

(1) HOApApTpTpCpApTpGpTpGpTpTOH (A1)

(2) HOApCpTpGpCpCpApGpGpApCpCpCpApTOH (A2)

(3) HOApTpGpTpApApApApGpApApGpCpApGOH (A3)

(4) HOTpGpGpCpApGpTpApApCpApCpApTpGOH (A4)

(5) HOTpTpTpApCpApTpApTpGpGpGpTpCpCOH (A5)

(6) HOApApGpGpTpTpTpTpCpTpGpCpTpTpCpTOH (A6)

(7) HOApApApApCpCpTpTpApApGpApApApTpAOH (B1)

(8) HOCpTpTpTpApApTpGpCpApGpGpTpCpAOH (B2)

(9) HOTpTpCpApGpApTpGpTpApGpCpGpGpAOH (B3)

(10) HOApTpTpApApApGpTpApTpTpTpCpTpTOH (B4)

(11) HOApTpCpTpGpApApTpGpApCpCpTpGpCOH (B5)

(12) HOTpTpCpCpApTpTpApTpCpCpGpCpTpApCOH (B6)

(13) HOTpApApTpGpGpApApCpTpCpTpTpTpTpCOH (C1)

(14) HOTpTpApGpGpCpApTpTpTpTpGpApApGOH (C2)

(15) HOApApTpTpGpGpApApApGpApGpGpApGOH (C3)

(16) HOTpGpCpCpTpApApGpApApApApGpApGOH (C4)

(17) HOTpCpCpApApTpTpCpTpTpCpApApApAOH (C5)

(18) HOCpTpGpTpCpApCpTpCpTpCpCpTpCpTpTOH (C6)

(19) HOApGpTpGpApCpApGpApApApApApTpAOH (D1)

(20) HOApTpGpCpApGpApGpCpCpApApApTpTOH (D2)

(21) HOGpTpCpTpCpCpTpTpTpTpApCpTpTOH (D3)

(22) HOCpTpCpTpGpCpApTpTpApTpTpTpTpTOH (D4)

(23) HOApGpGpApGpApCpApApTpTpTpGpGOH (D5)

(24) HOApApApGpCpTpTpGpApApGpTpApApAOH (D6)

(25) HOCpApApGpCpTpTpTpTpCpApApApApAOH (E1)

(26) HOCpTpTpTpApApGpGpApTpGpApCpCpAOH (E2)

(27) HOGpApGpCpApTpCpCpApApApApGpApGOH (E3)

(28) HOCpCpTpTpApApApGpTpTpTpTpTpGpAOH (E4)

(29) HOGpGpApTpGpCpTpCpTpGpGpTpCpApTOH (E5)

(30) HOTpCpTpCpCpApCpApCpTpCpTpTpTpTOH (E6)

(31) HOTpGpTpGpGpApGpApCpCpApTpCpApAOH (F1)

(32) HOGpGpApApGpApCpApTpGpApApTpGpTOH (F2)

(33) HOCpApApGpTpTpTpTpTpCpApApTpApGOH (F3)

(34) HOTpGpTpCpTpTpCpCpTpTpGpApTpGpGOH (F4)

(35) HOApApApApCpTpTpGpApCpApTpTpCpAOH (F5)

(36) HOTpTpTpTpGpTpTpGpCpTpApTpTpGpAOH (F6)

(37) HOCpApApCpApApApApApGpApApApCpGOH (G1)

(38) HOTpGpApTpGpApCpTpTpCpGpApApApAOH (G2)

(39) HOGpCpTpGpApCpTpApApTpTpApTpTpCOH (G3)

(40) HOApGpTpCpApTpCpApCpGpTpTpTpCpTOH (G4)

(41) HOTpApGpTpCpApGpCpTpTpTpTpCpGpAOH (G5)

(42) HOCpApGpTpTpApCpCpGpApApTpApApTOH (G6)

(43) HOGpGpTpApApCpTpGpApCpTpTpGpApAOH (H1)

(44) HOTpGpTpCpCpApApCpGpCpApApApGpCOH (H2)

(45) HOApApTpApCpApTpGpApApCpTpCpApTpCOH (H3)

(46) HOGpTpTpGpGpApCpApTpTpCpApApGpTOH (H4)

(47) HOCpApTpGpTpApTpTpGpCpTpTpTpGpCOH (H5)

(48) HOCpApApGpTpGpApTpGpGpCpTpGpApAOH (I1)

(49) HOCpTpGpTpCpGpCpCpApGpCpApGpCpTOH (I2)

(50) HOApApApApCpApGpGpGpApApGpCpGpAOH (I3)

(51) HOGpGpCpGpApCpApGpTpTpCpApGpCpCOH (I4)

(52) HOCpCpTpGpTpTpTpTpApGpCpTpGpCpTOH (I5)

(53) HOCpTpApCpGpTpTpTpTpCpGpCpTpTpCOH (I6)

(54) HOApApApCpGpTpApGpTpCpApGpApTpGpCOH (J1)

(55) HOTpGpTpTpTpCpApApGpGpTpCpGpApAOH (J2)

(56) HOGpApGpCpApTpCpCpCpApGpTpApApGOH (J3)

(57) HOTpGpApApApCpApGpCpApTpCpTpGpAOH (J4)

(58) HOGpApTpGpCpTpCpTpTpCpGpApCpCpTOH (J5)

(59) HOGpApTpCpCpTpTpApCpTpGpGOH (J6)

(60) HOTpGpTpGpTpApApTpGpApTpApGOH (L1)

(61) HOTpApCpApCpApCpTpCpTpTpTpTOH (L2)

(62) HOGpApTpCpCpCpTpApTpCpApTOH (L3)

<u>Example 3</u>

Synthesis of oligonucleotides (A, B, C, D, E, F, G, H, I, J, K, L, M and N):

Following oligonucleotides were prepared by a similar manner to that of Example 1.

(1) HOApApTpTpTpGpCpCpGpApCpAOH (A)

(2) HOCpGpTpTpApTpGpApTpGpTpCpGpGpCpAOH (B)

(3) HOTpCpApTpApApCpGpGpTpTpCpTpGpGpCOH (C)

(4) HOGpApApTpApTpTpTpGpCpCpApGpApApCOH (D)

(5) HOApApApTpApTpTpCpTpGpApApApTpGpAOH (E)

(6) HOTpCpApApCpApGpCpTpCpApTpTpTpCpAOH (F)

(7) HOGpCpTpGpTpTpGpApCpApApTpTpApApTOH (G)

(8) HOGpTpTpCpGpApTpGpApTpTpApApTpTpGOH (H)

(9) HOCpApTpCpGpApApCpTpApGpTpTpApApCOH (I)

(10) HOGpCpGpTpApCpTpApGpTpTpApApCpTpAOH (J)

(11) HOTpApGpTpApCpGpCpApApGpTpTpCpApCOH (K)

(12) HOCpTpTpTpTpTpApCpGpTpGpApApCpTpTOH (L)

(13) HOGpTpApApApApApGpGpGpTpApTpCpGOH (M)

(14) HOApApTpTpCpGpApTpApCpCOH (N)

Example 4

Synthesis of oligonucleotides (SA, SB, SC, SD, SE, SF, SG, and SH):

Following oligonucleotides were prepared by a similar manner to that of Example 1.

(1) HOApApTpTpCpApTpGpGpCpTOH (SA)

(2) HOGpGpTpTpGpTpApApGpApApCpTpTpCpTOH (SB)

(3) HOTpTpTpGpGpApApGpApCpTpTpTOH (SC)

(4) HOCpApCpTpTpCpGpTpGpTpTpGpApTpApGOH (SD)

(5) HOTpTpApCpApApCpCpApGpCpCpApTpGOH (SE)

(6) HOCpCpApApApApGpApApGpTpTpCOH (SF)

(7) HOCpGpApApGpTpGpApApApGpTpCpTpTOH (SG)

(8) HOGpApTpCpCpTpApTpCpApApCpAOH (SH)

<u>Example 5</u>

Preparation of IFN gene:

Ligation of chemically synthesized oligonucleotides:

Aliquots of each oligonucleotides (A2-L2) (0.4 nM) were phosphorylated with 2.5 units of T4 polynucleotide kinase in 40 µl of a solution containing 50 mM Tris-HCl (pH 7.6), 20 mM DTT, 50 µg/ml BSA, 1 mM spermidine, 10 mM $MgCl_2$ and 2 mM ATP for 3 hours at 37°C. After the reaction was completed, the enzyme in the reaction

mixture was deactivated by incubation at 100°C for 5 minutes. Ligation of the phosphorylated oligonucleotides and two oligonucleotides (A1 and L3) was carried out as shown in Fig. 3 to give firstly fragment six blocks and ultimately the structural protein LH gene (236 bp) for cloning. Ligation was carried out with T4 DNA ligase (5 units) in a solution containing 50 mM ATP (1 μl) for 5 hours at 16°C. The ligation products of oligonucleotides in each step were identified by staining with ethidium bromide following electroelution on a 2-16% gradient PAGE in Tris-EDTA buffer.

Example 6

Molecular cloning of the IFN LH gene:

Plasmid pBR322 was digested with BamHI and EcoRI restriction endonucleases. Reaction was terminated by heating at 65° C for 5 minutes and the fragments separated by electrophoresis on a 0.5% agarose gel. The 3985 bp large fragment from pBR322 was recovered and ligated with T4 DNA ligase for 18 hours at 12° C to the 236 bp structural protein LH gene. The ligated mixture was transformed into E. coli HB101 by Kushner's method and ampicillin resistant transformants were selected on the plate containing tetracycline (25 μg/ml). Plasmid DNA isolated from the clone resistant to ampicillin and sensitive to tetracycline was digested with EcoRI and BamHI and compared with appropriate size markers. The expected 236 bp structural protein LH gene fragment was generated. The plasmid (pLH107) obtained from E. coli HB101 transformant was characterized by restriction enzyme analysis to have a structural protein LH (236bp).

Example 7

Preparation of IFN RH gene:

Aliquots of each oligonucleotides (D3 to J5) (0.4 mM) were phosphorylated as described in Example 5. Ligation of the phosphorylated oligonucleotids and a oligonucleotide J6 was carried out as shown in Fig. 4 to give firstly fragment 7 Blocks and ultimately the IFN RH gene (270 bp). The IFN RH gene was partially purified by preparative PAGE, and was used for the cloning of IFN gene.

Example 8

Molecular cloning of IFN gene:

The plasmid (pLH107) obtained in Example 6 was digested with HindIII and BamHI. The larger fragment was recovered by preparative agarose gel electrophoresis, and then was ligated with IFN RH gene (270 bp) described in Example 5 in the presence of T4 DNA ligase. The ligation mixture was transformed into E. coli HB101. The plasmid (pF-3) obtained from the transformant was characterized by EcoRI and BamHI digestion to have the IFN gene (450 bp).

Example 9

Construction of the tryptophan promoter II gene:

Each oligonucleotides (B to SG) of block I', II', III' and IV' were phosphorylated with T4 polynucleotide kinase and then ligated with T4 DNA ligase as described

above. These blocks (I' to IV') and unphosphorylated oligonucleotides (A and SH) were condensed successively. The last ligation product was purified by preparative 7.5% PAGE to give the 163 bp trp promoter II gene.

Example 10

Cloning of the trp promoter II gene:

Trp promoter II gene constructed in Example 9 was ligated with EcoRI, BamHI fragment of pBR322 and then E. coli HB101 was transformed with the ligation product. The plasmid obtained from the transformant of $^{R}$Amp and $^{S}$Tet was digested with HpaI to confirm a band (4.1 kbp), and then digested with BamHI to confirm a band of 90 bp on PAGE. Moreover, the fragment of 56 bp by EcoRI-BamHI digestion was confirmed by the comparison with size marker on PAGE. This plasmid was named pTrpEB7 and used construction of expression vector.

Example 11

Construction of IFN LH expression vector (pγtrp):

Trp promoter II vector (pTrpEB7) prepared in Example 10 was digested with EcoRI and BamHI to give a large fragment (4.1 kbp) by preparative agarose gel electrophoresis. This fragments was ligated with IFN gene prepared from a plasmid pLH107 by EcoRI and BamHI digestion.. The ligated mixture was transformed into E. coli HB101 to give ampicillin resistant and tetracycline sensitive transformants. The plasmid (pγtrp) obtained from the transformant was digested with EcoRI and BamHI to confirm the IFN gene (236 bp) on 7.5% PAGE. The E. coli HB101 containing plasmid pγtrp was named E. coli F-9 and deposited with Fermentation Research Institute Agency

of Industrial Science and Technology (1-3, Higashi 1 chome Yatabe-machi Tsukuba-gun Ibaraki-ken 305, Japan) under deposit number of FERM P-7863 on September 20, 1984, and then converted to Budapest Treaty route of the same depository on September 12, 1985 under the new deposit number of FERM BP-905.

Example 12

An overnight culture of *E. coli* F-9 (which is *E. coli* HB101 containing plasmid pγtrp) (FERM P-7863) in L broth containing 50μg/ml ampicillin was diluted 1:20 in M9 medium containing 0.2% glucose, 0.5% casamino acid (acid-hydrolyzed casein), 50 μg/ml vitamin B1 and 25 μg/ml ampicillin. ß-Indole acylic acid was added to a final concentration of 10μg/ml when $A_{600}$ was 0.5. Then the cells were incubated for 2 hours and collected by centrifugation (5 krpm, 4°C, 5 minutes).

Example 13

Construction of IFN expression vector (pγtrp):

Trp promoter vector (pTrpEB7) prepared in Example 10 was digested with EcoRI and BamHI to give a large fragment (4.1 kbp) by preparative agarose gel electrophoresis. This fragment was ligated with ING gene prepared from a plasmid pF-3 by EcoRI and BamHI digestion. The ligated mixture was transformed into *E. coli* HB101 using the usual manner (Kusher's procedure). The plasmid (pγtrp) obtained from the transformant was characterized by following restriction enzyme analysis, PstI-HapaI (825 bp), PatI-EcoRI (859 bp), HpaI-BamHI (484

bp), HpaI-HindIII (214 bp), EcoRI-HindIII (180 bp), EcoRI-BamHI (450 bp), and HindIII-BamHI (270 bp).

Example 14

The sequencing of IFN gene was carried out using M13 didexoy method developed by F. Sanger. M13 vecter (M13mp8RT) was digested with EcoRI and BamHI. The fragment (7.2 kbp) was recovered by Sephacryl S 1000 gel filtration, and then was ligated with the IFN gene (450 bp), obtained from plasmlid pγtrp by EcoRI and BamHI digestion, in the presence of T4 DNA ligase. The ligated mixture was added to the complete cells (0.3 ml) of *E. coli* JM103, prepared by usual manner, and then stood for 1 hour at 4° C. After the heat shock (for 90 seconds at 42° C), to the mixture the incubation buffer (0.2 ml) containing 2% Xgal (40 μl) and 100 mM IPTG (40 μl) was added. After addition of top agar (3 ml) the mixture was spreaded on agar plates, and incubated overnight at 37° C. The resulting single white plaque was incubated in incubation buffer (1 ml) overnight at 37° C. After the centrifugation of the cells, to the supernatant PEG 6000 and NaCl were added to precipitate phage particles. The precipitate was collected by centrifugation and suspended into TE solution (150 ml), and followed by phenol extraction and ethanol precipitation. From the crude single strand DNA (ssDNA), the ssDNA of M13mp8 was removed by preparative agarose gel electrophoresis. The concentration of the ssDNA was adjusted to 500 ng/μl. The ssDNA was analyzed according to the manual of M13 didexoy method.

The results of sequencing were agreed with the designed IFN gene.

Example 15

Expression of IFN gene in E. coli and the characterization:

An overnight culture of E coli HB101 containing plasmid pγtrp in L broth containing 50μg/ml ampicillin was diluted 1:20 in M9 medium containing 0.2 % glucose, 0.5% casamino acid, 50 μg/ml thiamine, and 25 μg/ml ampicillin, and then incubated at 37° C. β-Indole acrylic acid was added to a final concentration of 10 μg/ml when $A_{600}$ was 0.5. Then the cells were incubated for 3 hours and collected by centrifugation (8 kbp, 4° C, 10 minutes). The cells were suspended in 10 mM PBS-EDTA, and opened by sonicaiton at 0° C. The suspended mixture was centrifuged, the supernatant was examined by the assay of anti-virus activity to detect the activity of IFN ($1.3 \times 10^6$ IU/ml culture). Also a band of IFN was detected at a position of about 17.1 k dalton on the SDS-PAGE. The activity of the obtained IFN ($4.2 \times 10^5$ IU/ml) decreased until 42 IU/ml by adjusting at pH 2.0 with 1N HCl. By the above evidence the production of IFN was characterized.

Example 16

Bioassay of IFN:

FL cells of $4 \times 10^5$ cells/ml in Eagles' MEM containing 5% FBS were added in 96 well microplate with 0.1 ml portion. The cells were incubated at 37°C for 3 hours in the 5% $CO_2$ incubator, and then the incubation buffer was changed with Eagle's MEM containing 2% FBS. To the cells the dilution solution containing IFN was added and incubated at 37°C overnight in the 5% $CO_2$ incubator.

After removing the IFN solution the incubation buffer was changed with Eagle's MEM, and then Sindvis virus ($10^6$ ffu/ml, 0.1 ml) siluted with Eagle's MEM contining 0.05% BSA was added. After incubation for 24 hours at 37° C in 3% $CO_2$ incubator, the cells were fixed with 10% formalin, and then colored with 0.1% crystal violet solution. Activity (IU/ml) of IFN was determined from the observed value (U/ml) based on NIH international reference standard (LeuHuIFNα, G023-901-527).

What we claim is:

1. A gene coding for γ-interferon (herinafter referred to as IFN) in which the gene comprises the following sequence:

```
                                    1
                    EcoRI Met Cys Tyr Cys Gln Asp Pro Tyr
    Coding:      5'-AATTC-ATG-TGT-TAC-TGC-CAG-GAC-CCA-TAT-
    Noncoding:      3'-G-TAC-ACA-ATG-ACG-GTC-CTG-GGT-ATA-

      10                                      20
Val Lys Glu Ala Glu Asn Leu Lys Lys Tyr Phe Asn Ala Gly
GTA-AAA-GAA-GCA-GAA-AAC-CTT-AAG-AAA-TAC-TTT-AAT-GCA-GGT-
CAT-TTT-CTT-CGT-CTT-TTG-GAA-TTC-TTT-ATG-AAA-TTA-CGT-CCA-

                                  30
His Ser Asp Val Ala Asp Asn Gly Thr Leu Phe Leu Gly Ile
CAT-TCA-GAT-GTA-GCG-GAT-AAT-GGA-ACT-CTT-TTC-TTA-GGC-ATT-
GTA-AGT-CTA-CAT-CGC-CTA-TTA-CCT-TGA-GAA-AAG-AAT-CCG-TAA-

                  40
Leu Lys Asn Trp Lys Glu Glu Ser Asp Arg Lys Ile Met Gln
TTG-AAG-AAT-TGG-AAA-GAG-GAG-AGT-GAC-AGA-AAA-ATA-ATG-CAG-
AAC-TTC-TTA-ACC-TTT-CTC-CTC-TCA-CTG-TCT-TTT-TAT-TAC-GTC-

 50                               HindIII 60
Ser Gln Ile Val Ser Phe Tyr Phe Lys Leu Phe Lys Asn Phe
AGC-CAA-ATT-GTC-TCC-TTT-TAC-TTC-AAG-CTT-TTC-AAA-AAC-TTT-
TCG-GTT-TAA-CAG-AGG-AAA-ATG-AAG-TTC-GAA-AAG-TTT-TTG-AAA-

                          70
Lys Asp Asp Gln Ser Ile Gln Lys Ser Val Glu Thr
AAG-GAT-GAC-CAG-AGC-ATC-CAA-AAG-AGT-GTG-GAG-ACC-
TTC-CTA-CTG-GTC-TCG-TAG-GTT-TTC-TCA-CAC-CTC-TGG-
```

80

Ile Lys Glu Asp Met Asn Val Lys Phe Phe Asn Ser Asn Lys
ATC-AAG-GAA-GAC-ATG-AAT-GTC-AAG-TTT-TTC-AAT-AGC-AAC-AAA-
TAG-TTC-CTT-CTG-TAC-TTA-CAG-TTC-AAA-AAG-TTA-TCG-TTG-TTT-

90 100

Lys Lys Arg Asp Asp Phe Glu Lys Leu Thr Asn Tyr Ser Val
AAG-AAA-CGT-GAT-GAC-TTC-GAA-AAG-CTG-ACT-AAT-TAT-TCG-GTA-
TTC-TTT-GCA-CTA-CTG-AAG-CTT-TTC-GAC-TGA-TTA-ATA-AGC-CAT-

110

Thr Asp Leu Asn Val Gln Arg Lys Ala Ile His Glu Leu Ile
ACT-GAC-TTG-AAT-GTC-CAA-CGC-AAA-GCA-ATA-CAT-GAA-CTC-ATC-
TGA-CTG-AAC-TTA-CAG-GTT-GCG-TTT-CGT-TAT-GTA-CTT-GAG-TAG-

120 130

Gln Val Met Ala Glu Leu Ser Pro Ala Ala Lys Thr Gly Lys
CAA-GTG-ATG-GCT-GAA-CTG-TCG-CCA-GCA-GCT-AAA-ACA-GGG-AAG-
GTT-CAC-TAC-CGA-CTT-GAC-AGC-GGT-CGT-CGA-TTT-TGT-CCC-TTC-

140

Arg Lys Arg Ser Gln Met Leu Phe Gln Gly Arg Arg Ala Ser
CGA-AAA-CGT-AGT-CAG-ATG-CTG-TTT-CAA-GGT-CGA-AGA-GCA-TCC-
GCT-TTT-GCA-TCA-GTC-TAC-GAC-AAA-GTT-CCA-GCT-TCT-CGT-AGG-

Gln stop BamHI
CAG-TAA-G
GTC-ATT-CCTAG

or subunit thereof.

2. A process for the production of a gene as claimed in claim 1 which comprises linking a gene coding for IFN LH with a gene coding for IFN-RH.

3. A gene which codes for the expression of IFN LH, wherein IFN LH consists of 73 amino acids in the following sequence.

```
    1                                          10
     Cys-Tyr-Cys-Gln-Asp-Pro-Tyr-Val-Lys-Glu-Ala-Glu-

                                   20
Asn-Leu-Lys-Lys-Tyr-Phe-Asn-Ala-Gly-His-Ser-Asp-Val-

                  30
Ala-Asp-Asn-Gly-Thr-Leu-Phe-Leu-Gly-Ile-Leu-Lys-Asn-

     40                                        50
Trp-Lys-Glu-Glu-Ser-Asp-Arg-Lys-Ile-Met-Gln-Ser-Gln-

                                        60
Ile-Val-Ser-Phe-Tyr-Phe-Lys-Leu-Phe-Lys-Asn-Phe-Lys-

                       70
Asp-Asp-Gln-Ser-Ile-Gln-Lys-Ser-Val
```

or subunit thereof.

4. A gene of Claim 3, in which the gene comprises the following sequence:.

```
                                    1
                     EcoRI Met Cys Tyr Cys Gln Asp Pro Tyr
     Coding:    5'-AATTC-ATG-TGT-TAC-TGC-CAG-GAC-CCA-TAT-
     Noncoding:      3'-G-TAC-ACA-ATG-ACG-GTC-CTG-GGT-ATA-
```

```
           10                                        20
Val Lys Glu Ala Glu Asn Leu Lys Lys Tyr Phe Asn Ala Gly
GTA-AAA-GAA-GCA-GAA-AAC-CTT-AAG-AAA-TAC-TTT-AAT-GCA-GGT-
CAT-TTT-CTT-CGT-CTT-TTG-GAA-TTC-TTT-ATG-AAA-TTA-CGT-CCA-

                                   30
His Ser Asp Val Ala Asp Asn Gly Thr Leu Phe Leu Gly Ile
CAT-TCA-GAT-GTA-GCG-GAT-AAT-GGA-ACT-CTT-TTC-TTA-GGC-ATT-
GTA-AGT-CTA-CAT-CGC-CTA-TTA-CCT-TGA-GAA-AAG-AAT-CCG-TAA-

                   40
Leu Lys Asn Trp Lys Glu Glu Ser Asp Arg Lys Ile Met Gln
TTG-AAG-AAT-TGG-AAA-GAG-GAG-AGT-GAC-AGA-AAA-ATA-ATG-CAG-
AAC-TTC-TTA-ACC-TTT-CTC-CTC-TCA-CTG-TCT-TTT-TAT-TAC-GTC-

 50                                HindIII 60
Ser Gln Ile Val Ser Phe Tyr Phe Lys Leu Phe Lys Asn Phe
AGC-CAA-ATT-GTC-TCC-TTT-TAC-TTC-AAG-CTT-TTC-AAA-AAC-TTT-
TCG-GTT-TAA-CAG-AGG-AAA-ATG-AAG-TTC-GAA-AAG-TTT-TTG-AAA-

                             70
Lys Asp Asp Gln Ser Ile Gln Lys Ser Val Stop Stop BamHI
AAG-GAT-GAC-CAG-AGC-ATC-CAA-AAG-AGT-GTG-TAA-TGA-TAG
TTC-CTA-CTG-GTC-TCG-TAG-GTT-TTC-TCA-CAC-ATT-ACT-ATCCTAG
```

or subunit thereof.

5. A process for the production of a gene as claimed in claim 3 or claim 4 which comprises the hybridization and ligation of a number of the corresponding oligonucleotide blocks.

6. A gene which codes for the expression of IFN RH, wherein IFN RH consists of 87 amino acids in the following sequence.

60 70
Phe-Lys-Asn-Phe-Lys-Asp-Asp-Gln-Ser-Ile-Gln-Lys-

80
Ser-Val-Glu-Thr-Ile-Lys-Glu-Asp-Met-Asn-Val-Lys-Phe-

90
Phe-Asn-Ser-Asn-Lys-Lys-Lys-Arg-Asp-Asp-Phe-Glu-Lys-

100 110
Leu-Thr-Asn-Tyr-Ser-Val-Thr-Asp-Leu-Asn-Val-Gln-Arg-

120
Lys-Ala-Ile-His-Glu-Leu-Ile-Gln-Val-Met-Ala-Glu-Leu-

130
Ser-Pro-Ala-Ala-Lys-Thr-Gly-Lys-Arg-Lys-Arg-Ser-Gln-

140 146
Met-Leu-Phe-Gln-Gly-Arg-Arg-Ala-Ser-Gln

or subunit thereof.

7. A gene of claim 6, in which the gene comprises the following sequence:

60

HindIII Phe Lys

Coding: 5'-AG-CTT-TTC-AAA-

Noncoding: 3'-A-AAG-TTT-

70

Asn Phe Lys Asp Asp Gln Ser Ile Gln Lys Ser Val Glu Thr
AAC-TTT-AAG-GAT-GAC-CAG-AGC-ATC-CAA-AAG-AGT-GTG-GAG-ACC-
TTG-AAA-TTC-CTA-CTG-GTC-TCG-TAG-GTT-TTC-TCA-CAC-CTC-TGG-

80

Ile Lys Glu Asp Met Asn Val Lys Phe Phe Asn Ser Asn Lys
ATC-AAG-GAA-GAC-ATG-AAT-GTC-AAG-TTT-TTC-AAT-AGC-AAC-AAA-
TAG-TTC-CTT-CTG-TAC-TTA-CAG-TTC-AAA-AAG-TTA-TCG-TTG-TTT-

90 100

Lys Lys Arg Asp Asp Phe Glu Lys Leu Thr Asn Tyr Ser Val
AAG-AAA-CGT-GAT-GAC-TTC-GAA-AAG-CTG-ACT-AAT-TAT-TCG-GTA-
TTC-TTT-GCA-CTA-CTG-AAG-CTT-TTC-GAC-TGA-TTA-ATA-AGC-CAT-

110

Thr Asp Leu Asn Val Gln Arg Lys Ala Ile His Glu Leu Ile
ACT-GAC-TTG-AAT-GTC-CAA-CGC-AAA-GCA-ATA-CAT-GAA-CTC-ATC-
TGA-CTG-AAC-TTA-CAG-GTT-GCG-TTT-CGT-TAT-GTA-CTT-GAG-TAG-

120 130

Gln Val Met Ala Glu Leu Ser Pro Ala Ala Lys Thr Gly Lys
CAA-GTG-ATG-GCT-GAA-CTG-TCG-CCA-GCA-GCT-AAA-ACA-GGG-AAG-
GTT-CAC-TAC-CGA-CTT-GAC-AGC-GGT-CGT-CGA-TTT-TGT-CCC-TTC-

140

Arg Lys Arg Ser Gln Met Leu Phe Gln Gly Arg Arg Ala Ser
CGA-AAA-CGT-AGT-CAG-ATG-CTG-TTT-CAA-GGT-CGA-AGA-GCA-TCC-
GCT-TTT-GCA-TCA-GTC-TAC-GAC-AAA-GTT-CCA-GCT-TCT-CGT-AGG-

Gln stop BamHI
CAG-TAA-G
GTC-ATT-CCTAG

or subunit thereof.

8. A process for the production of a gene as claimed in claim 6 or claim 7 which comprises the hybridization and ligation of a number of the corresponding oligonucleotide blocks.

9. An expression vector containing a promoter gene and a gene coding for IFN.

10. A process for the production of an expression vector which comprises inserting a promoter gene and a gene coding for IFN into a plasmid.

11. A transformant containing an expression vector as defined in Claim 9.

12. A process for the production of a transformant which comprises transforming a host organism with an expression vector as defined in Claim 9.

13. A process for the production of IFN which comprises culturing a transformant as defined in Claim 11.

Fig. 1 Building up of oligonucleotides from smaller units by successive coupling reactions (1/4)

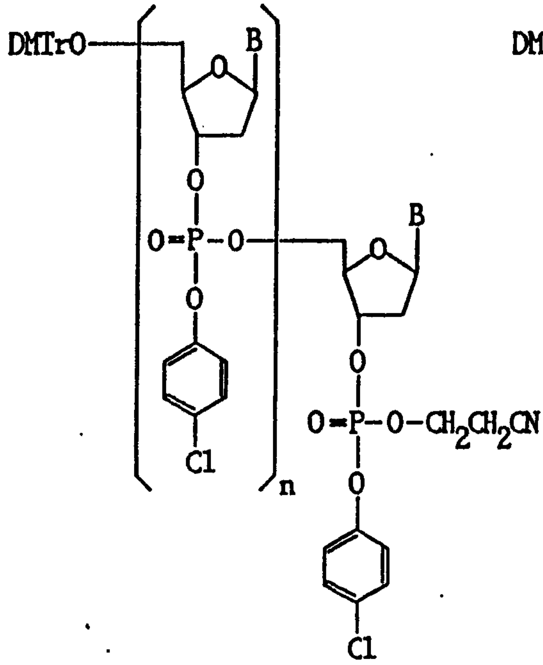

[DMTrO(Bpo)$_n$BpoCE]
( I )

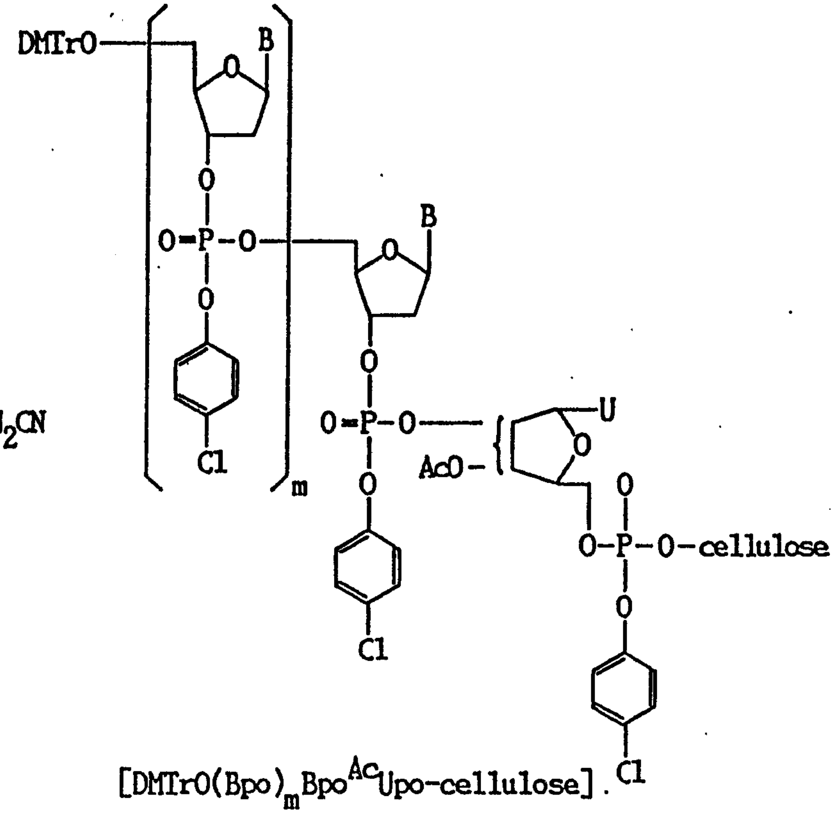

[DMTrO(Bpo)$_m$Bpo$^{Ac}$Upo-cellulose]
( II )

Fig. 1 (continued) Building up of oigonucleotides from smaller units by successive coupling reactions (2/4)

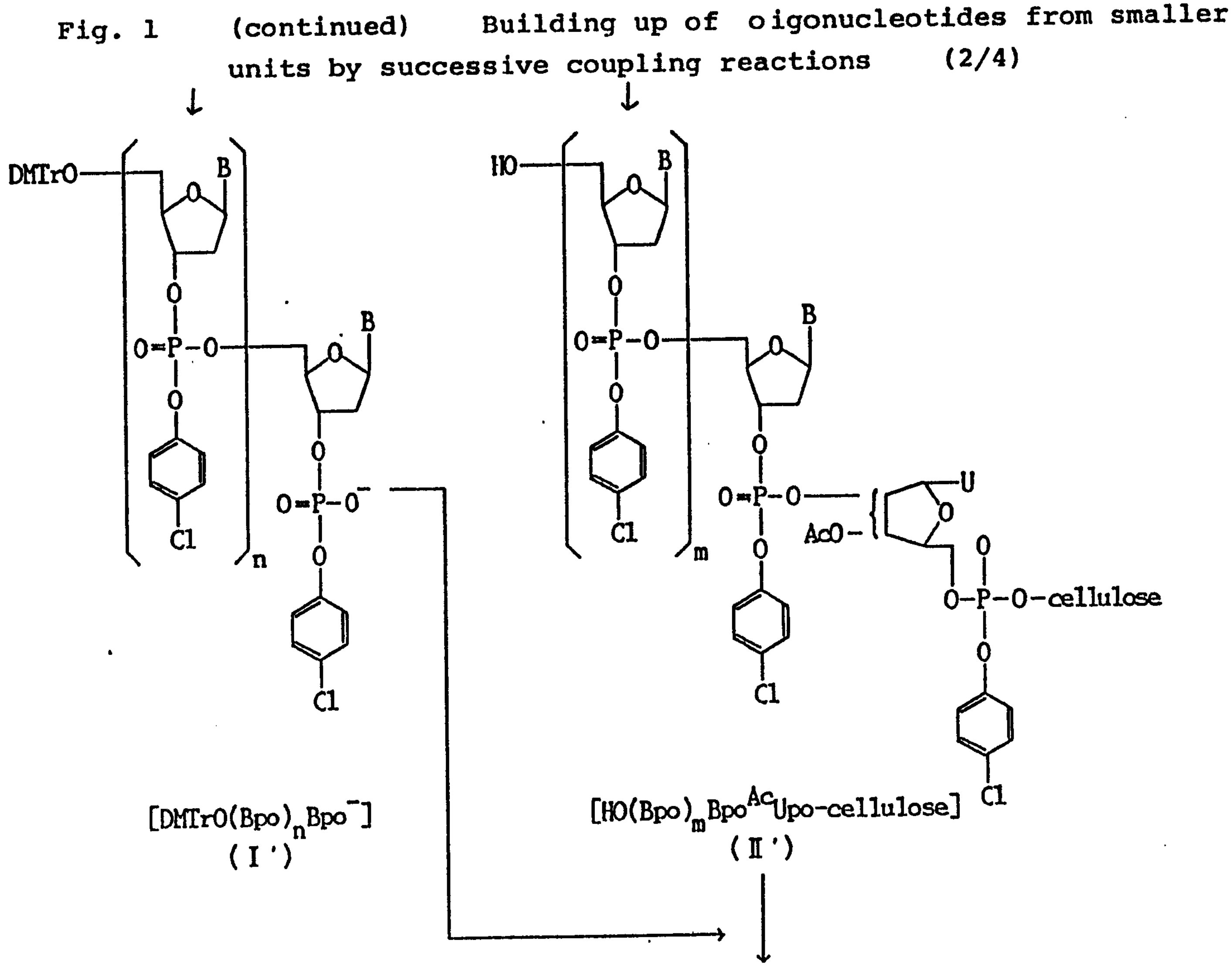

$[DMTrO(Bpo)_nBpo^-]$
(I')

$[HO(Bpo)_mBpo^{Ac}Upo\text{-}cellulose]$
(II')

Fig. 1 (continued) Building up of oligonucleotides from smaller units by successive coupling reactions (3/4)

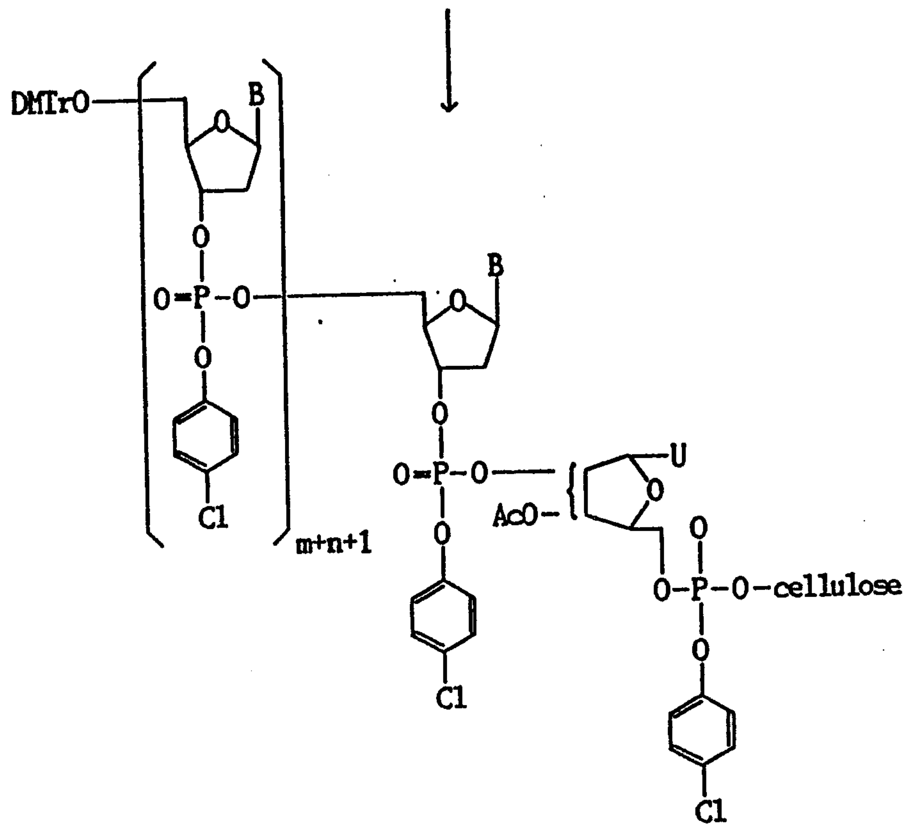

[DMTrO(Bpo)$_{m+n+1}$Bpo$^{Ac}$Upo-cellulose]

(III)

Fig. 1 (continued) Building up of oligonucleotides from smaller units by successive coupling reactions (4/4)

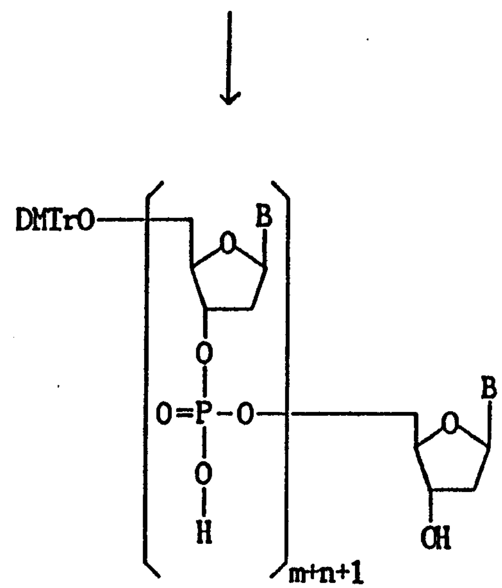

$[HO(Bp)_{m+n+1}BOH]$

(III')

Fig. 2 Flow chart of the preparation of the hexadecanucleotide
HOApTpCpApCpTpTpGpGpApTpGpApGpTpTOH (H6) (1/3)

DMTrOTpo$^{Ac}$Upo-cellulose
↓
HOTpo$^{Ac}$Upo-cellulose

DMTrOA$^{Bz}$poG$^{iB}$poTpo-CE
↓
DMTrOA$^{Bz}$poG$^{iB}$poTpo$^{-}$ →

↓
DMTrOA$^{Bz}$poG$^{iB}$poTpoTpo$^{Ac}$Upo-cellulose
↓
HOA$^{Bz}$po G$^{iB}$poTpoTpo$^{Ac}$Upo-cellulose

DMTrOA$^{Bz}$poTpoG$^{iB}$po-CE
↓
DMTrOA$^{Bz}$poTpoG$^{iB}$po$^{-}$ →

↓
DMTrOA$^{Bz}$poTpoG$^{iB}$poA$^{Bz}$poG$^{iB}$poTpoTpo$^{Ac}$Upo-cellulose
↓

Fig. 2 (continued) Flow chart of the preparation of the hexadecanucleotide HOApTpCpApCpTpTpGpGpApTpGpApGpTpTOH (H6) (2/3)

↓

$\mathrm{HOA^{Bz}poTpoG^{iB}poA^{Bz}poG^{iB}poTpoTpo^{Ac}Upo\text{-}cellulose}$

$\mathrm{DMTrOTpoG^{iB}poG^{iB}po\text{-}CE}$

↓

$\mathrm{HOTpoG^{iB}poG^{iB}po^{-}}$ ⟶

↓

$\mathrm{DMTrOTpoG^{iB}poG^{iB}poA^{Bz}poTpoG^{iB}poA^{Bz}poG^{iB}poTpoTpo^{Ac}Upo\text{-}cellulose}$

↓

$\mathrm{HOTpoG^{iB}poG^{iB}poA^{Bz}poTpoG^{iB}poA^{Bz}poG^{iB}TpoTpo^{Ac}Upo\text{-}cellulose}$

$\mathrm{DMTrOA^{Bz}poC^{Bz}poTpo\text{-}CE}$

↓

$\mathrm{HOA^{Bz}poC^{Bz}poTpo^{-}}$ ⟶

↓

$\mathrm{DMTrOA^{Bz}poC^{Bz}poTpoTpoG^{iB}poG^{iB}poA^{Bz}poTpoG^{iB}poA^{Bz}poG^{iB}poTpoTpo^{Ac}Upo\text{-}cellulose}$

↓

$\mathrm{HOA^{Bz}poC^{Bz}poTpoTpoG^{iB}poG^{iB}poA^{Bz}poTpoG^{iB}poA^{Bz}poG^{iB}poTpoTpo^{Ac}Upo\text{-}cellulose}$

Fig. 2 (continued) Flow chart of the preparation of the hexadecanucleotide HOApTpCpApCpTpTpGpGpApTpGpApGpTpTOH (H6) (3/3)

$DMTrOA^{Bz}poTpoC^{Bz}po\text{-}CE$

↓

$HOA^{Bz}poTpoC^{Bz}po^{-}$ ⟶

↓

$DMTrOA^{Bz}poTpoC^{Bz}poA^{Bz}poC^{Bz}poTpoTpoG^{iB}poG^{iB}poA^{Bz}poTpoG^{iB}poA^{Bz}poG^{iB}\text{-}$

$poTpoTpo^{Ac}Upo\text{-cellulose}$

↓

$HOA^{Bz}poTpoC^{Bz}poA^{Bz}poC^{Bz}poTpoTpoG^{iB}poG^{iB}poA^{Bz}poTpoG^{iB}poA^{Bz}poG^{iB}\text{-}$

$poTpoTpo^{Ac}Upo\text{-cellulose}$

↓

HOApTpCpApCpTpTpGpGpApTpGpApGpTpTOH

Fig. 3 Construction of IFN LH gene

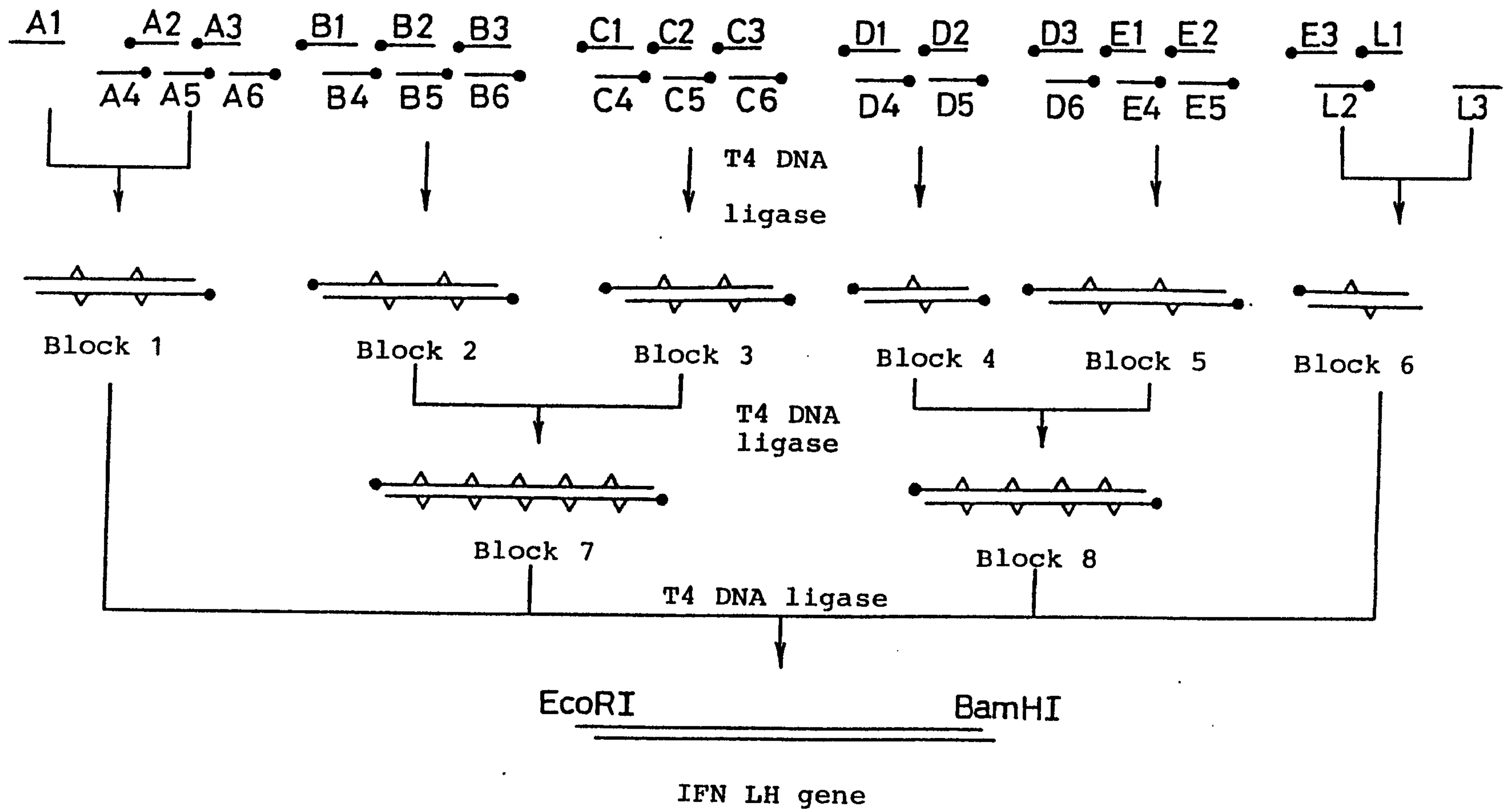

Fig. 4 Construction of IFN RH gene

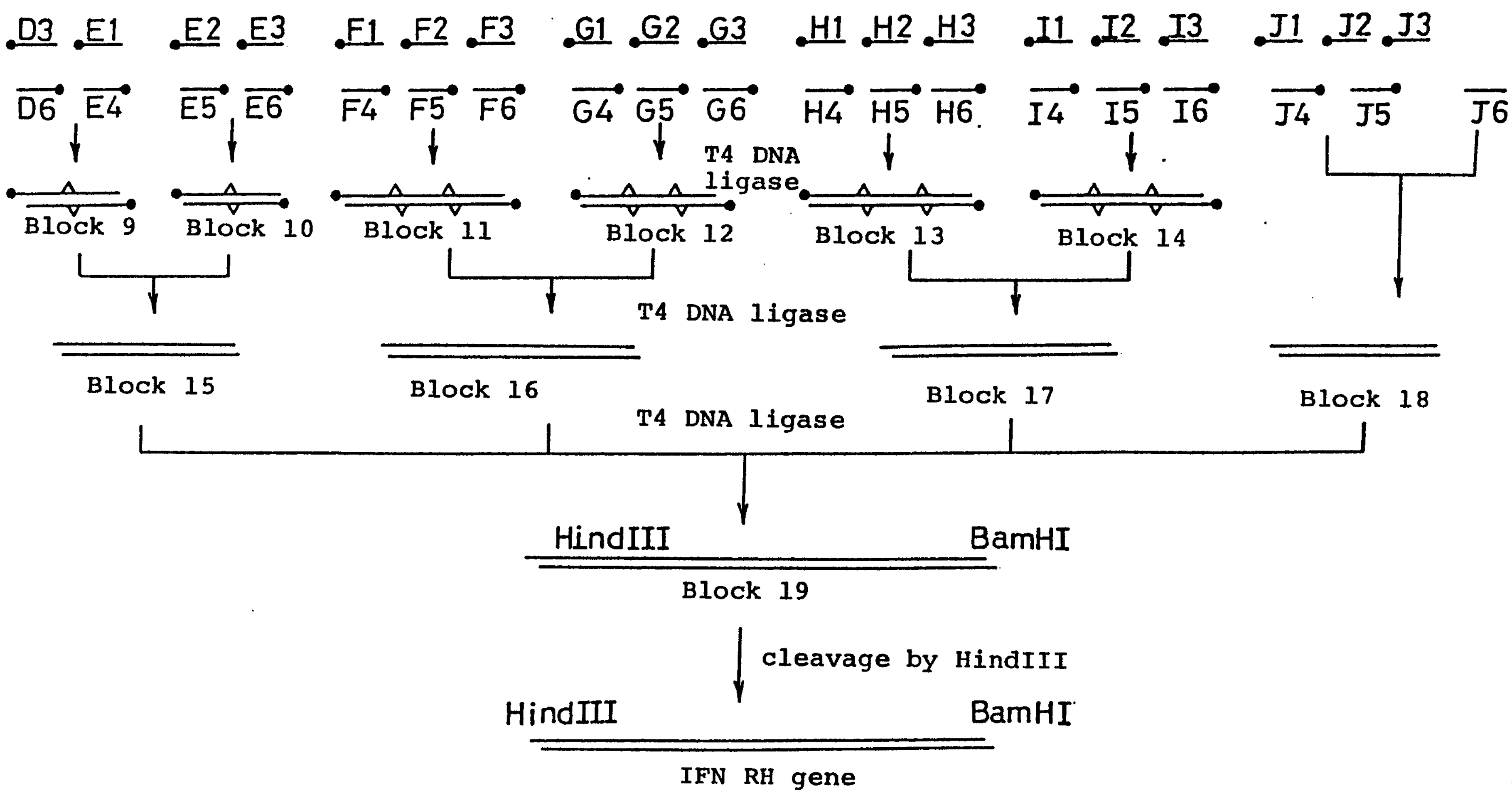

0176916

Fig. 5 Molecular cloning of IFN gene

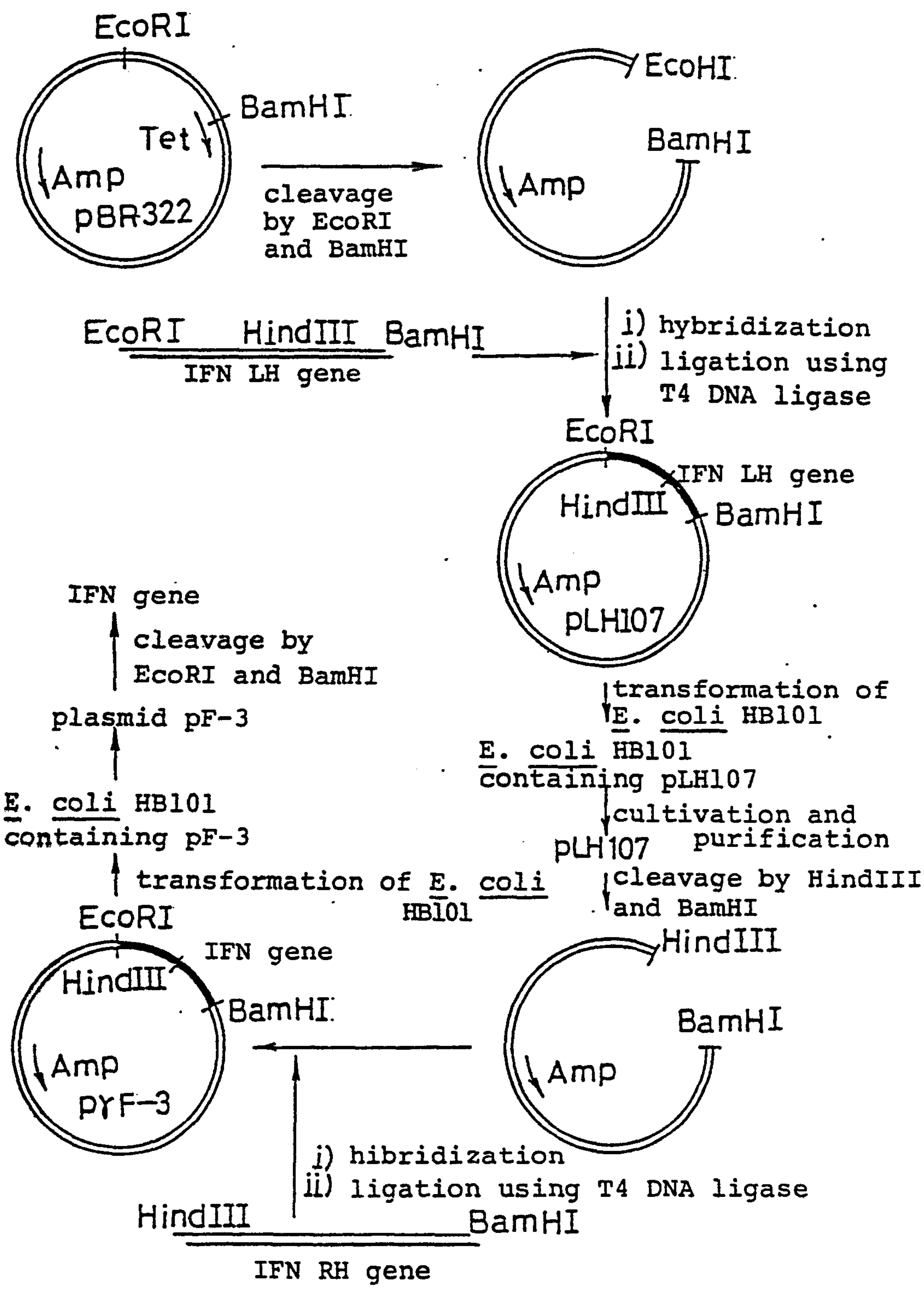

0176916

Fig. 6 Construction of synthetic trp promoter II gene

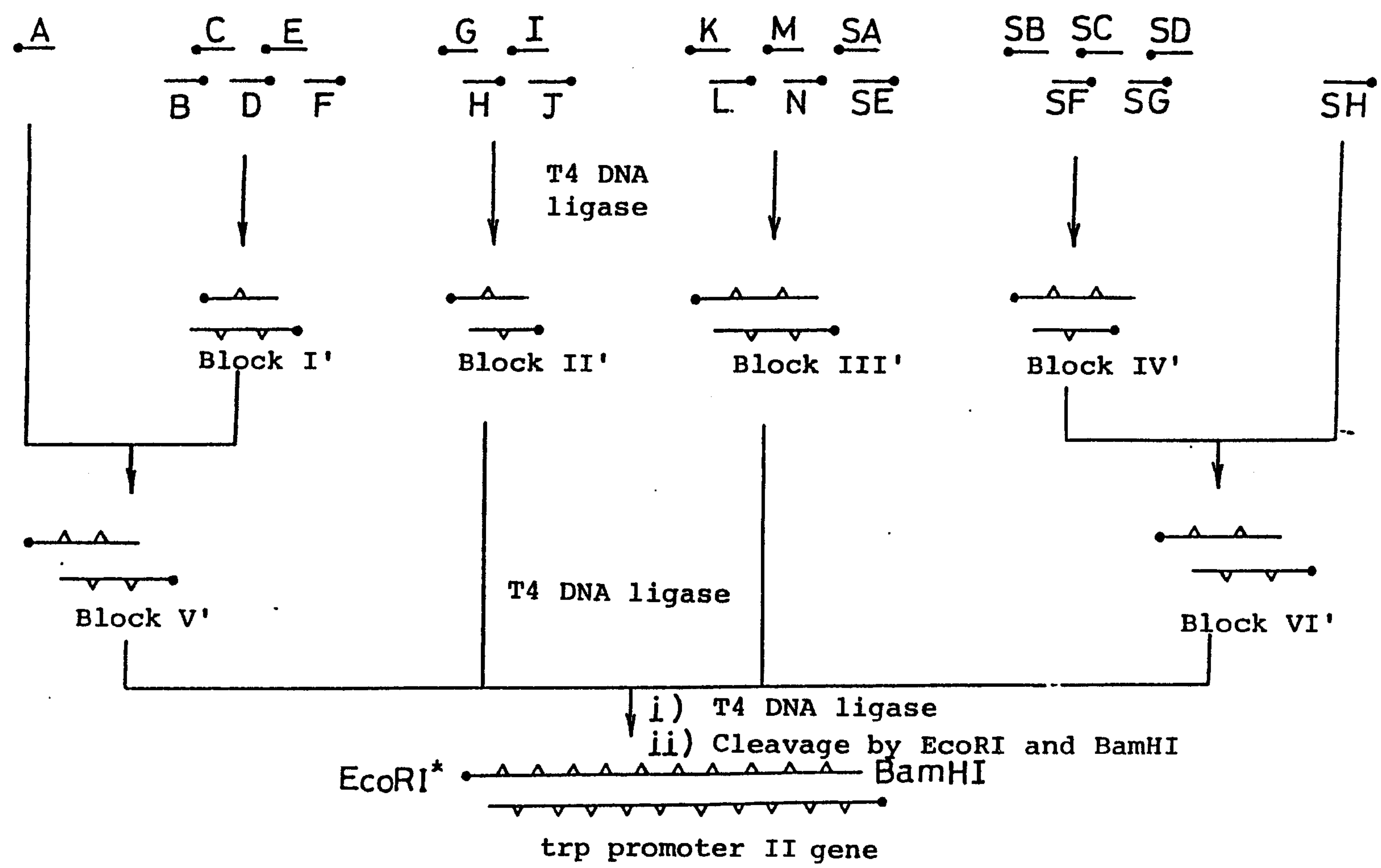

Fig. 7 Molecular cloning of synthetic trp promoter II

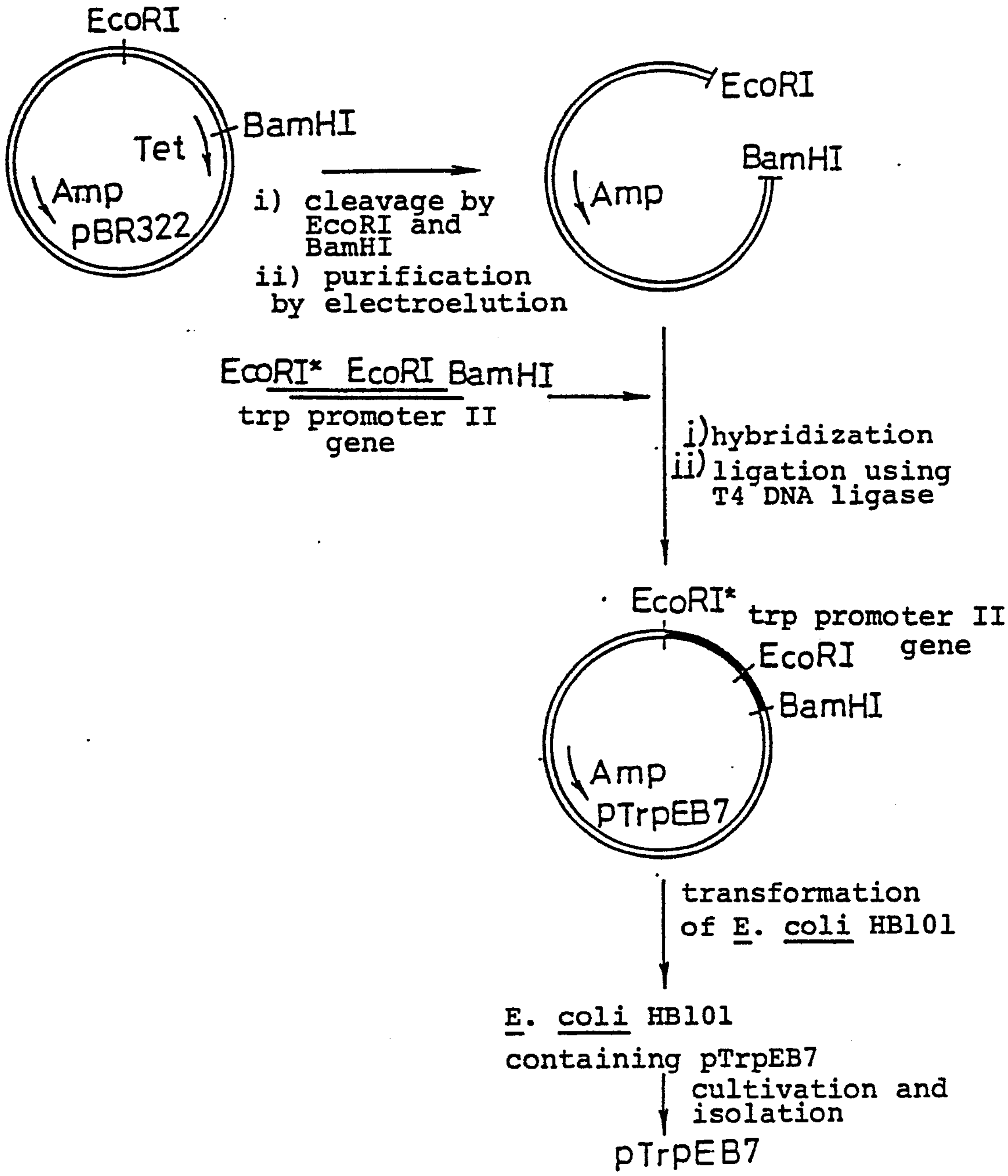

Fig. 8 Construction of recombinant plasmid